# EUROPEAN PATENT APPLICATION

(11) **EP 2 169 068 A1**
(43) Date of publication of application: **31.03.2010**
(21) Application number: 08290915.1
(22) Date of filing: 29.09.2008
(51) Int. Cl.: C12N 15/80, G01N 33/58, C12N 1/19

(54) **Bioluminescent fungi and their application in the study of fungal infections**

(71) Applicant: Institut Pasteur, 75015 Paris (FR); Leibniz Institute For Natural Product Research And Infection Biology E.V. Hans-Knöll-Institut (HKI), 07747 Jena (DE)
(72) Inventor: Brock, Matthias, 07751 Jena (DE); Ibrahim-Granet, Oumaïma, 92130 Issy Les Moulineaux (FR); Jouvion, Gregory, 75014 Paris (FR)
(74) Representative: Warcoin, Jacques

(57) **Abstract**

The present invention relates to a nucleic acid comprising a gene encoding a luciferase, which is under the control of a promoter for expression of said luciferase in a filamentous fungal host cell, such as an *Aspergillus fumigatus* host cell. Advantageously, the promoter is the glyceraldehyde-3-phosphate dehydrogenase promoter from *Aspergillus fumigatus*. The invention further relates to a transformed filamentous fungal host cell comprising the nucleic acid of the invention, and to an animal model using different immunosuppression regimens which has been infected with said filamentous fungal host cell. The invention also relates to methods for determining the virulence of filamentous fungal host cells, for studying a fungal infection, or for determining the antifungal activity of a compound.

## Description

The present invention relates to a nucleic acid comprising a gene encoding a luciferase, which is under the control of a promoter for expression of said luciferase in a filamentous fungal host cell, such as an *Aspergillus fumigatus* host cell. Advantageously, the promoter is the glyceraldehyde-3-phosphate dehydrogenase promoter from *Aspergillus fumigatus.* The invention further relates to a transformed filamentous fungal host cell comprising the nucleic acid of the invention, and to an animal model using different immunosuppression regimens which has been infected with said filamentous fungal host cell. The invention also relates to methods for determining the virulence of filamentous fungal host cells, for studying a fungal infection, or for determining the antifungal activity of a compound.

Fungal infections, also named mycoses, are diseases caused by fungi. Fungi are different from bacteria by the fact that they have a bigger structure. Their cellular wall contains cellulose (like in plants) or chitin (like in insects). Most of the fungi need oxygen and must take organic matter as nutrient. Indeed, contrary to plants, they are lacking in chlorophyll and thus they cannot carry out photosynthesis. A lot of fungi are edible, whereas others are used for manufacturing cheese, baker's yeast, beer or some antibiotics. Among the 100,000 classified fungal species, only approximately 180 of them are pathogenic for the human being. Among these pathogenic fungi, threre is the genus *Aspergillus,* which is important since it can cause a whole range of affections such as affection to the external ear, skin lesions, and ulcers classed as mycetomas.

*Aspergillus fumigatus* is one of the species of this genus that is responsible for a variety of infections in the immunocompetent as well as in the immunocompromised host. Moreover, *Aspergillus fumigatus* can cause a life-threatening invasive aspergillosis in immunocompromised patients (Latgé 1999). Patients at risk are mainly those, who have undergone organ transplantation or suffer from illnesses like leukaemia, neutropenia, HIV infection or cystic fibrosis (Denning 1994; Singh and Paterson 2005). In addition, several cases are reported in which persons after near-drowning developed invasive fungal infections (Leroy et al. 2006). Although infections caused by *A. fumigatus* are known since many years, the factors deriving from both sides, host and pathogen, which are involved in establishment and manifestation of the fungal infection, are hardly understood.

Several murine infection models have been established in recent years to identify virulence determinants which allow *A. fumigatus* to act as a pathogen (Sarfati et al. 2002). To study disease development, mice were formerly sacrificed at certain time points and investigated by histopathology with the drawback of organ selection from dead animals and the problem that only a snapshot of the infection process was observed.

It is well accepted that reduced immune defense mechanisms on the host side are generally required to allow fungal outgrowth. This is also reflected in infection models, in which high doses of conidia applied to the respiratory tract are well tolerated by immunocompetent individuals (Balloy et al. 2005; Dubourdeau et al. 2006; Montagnoli et al. 2006). In this immunocompetent situation it is believed that alveolar macrophages, dendritic cells and granulocytes form an insuperable barriere efficiently inactivating and removing all conidia. However, treatment of mice with immunosuppressive agents or by irradiation strongly reduces the amount of conidia required to cause invasive fungal growth (Liebmann et al. 2004; Stephens-Romero et al. 2005). Nowadays, two different kinds of immunosuppression are mainly used. The first model system uses neutropenic mice by application of the cytotoxine cyclophosphamide, which is very efficient in making mice susceptible for an infection but the interaction of the fungus with immune effector cells can hardly be studied. An alternative is the treatment of mice with high doses of corticosteroids, which reduces the ability of immune effector cells to kill conidia and outgrowing hyphae (Schaffner and Schaffner 1987) without depletion of one of the immune effector cell types. This model generally requires higher conidial loads than the former model but allows to study fungal interactions with the immune system (Dubourdeau et al. 2006).

For investigation of the development of invasive aspergillosis, conidia are generally applied intranasally or directly to the trachea by using either a suspension or an aerosol (Sheppard et al. 2006). This treatment is believed to resemble most closely the route of infection as it occurs in immunocompromised patients. However, although in humans infection generally starts at the site of the pulmonary tract, there are some reports on dissemination of fungal cells to the liver, brain and kidneys (Altiparmak et al. 2002; Gottfredsson and Steingrímsdóttir 2006; Hasan and Abuhammour 2006). Up to now, it was not clear, whether fungal dissemination also occurs in murine infection models. Histopathologic sections of infected mouse lungs clearly revealed inflammated lesions with a large fungal burden. Since crossing of mycelium through the cardiovascular membrane to the blood vessels can lead to pulmonary and cardiac infarction, it was assumed that mice, in general, die before fungal dissemination occurs. This implied that inhalation models are more or less restricted to the investigation of bronchopulmonar invasive aspergillosis rather than being suitable for the investigation of disseminated disease. In order to cause and study disseminated aspergillosis the route of infection is that *via* the tail vein of mice, in which conidia are transported by the blood stream and can, therefore, easily reach different organs. However, since primary infections of humans *via* the blood stream seems to occur only during a surgical procedure under none sterile conditions (Pasqualotto and Denning 2006), it remains questionable, whether this route of infection may be suitable for studying *Aspergillus* infections in general.

In recent years bioluminescence imaging (BLI) has evolved as a powerful technique to study the establishment and manifestation of infection by pathogens and has provided new insights into onset and dissemination of infections. The main advantage of this technique is that a real time monitoring of spatial and temporal infection can be achieved on single individual animals without the need to sacrifice the animal at a certain point of time, which only leads to a snapshot view of the infection process (Hutchens and Luker 2007). By using BLI new insights on the infection process can be gained accompanied by the possibility to significantly reduce the number of animals studied in each cohort.

BLI has been performed for several Gram-positive and Gram-negative bacteria (Glomski et al. 2007; Karsi and Lawrence 2007; Sjölinder and Jonsson 2007), as well as for different viruses (Hutchens and Luker 2007) and also for eucaryotic parasites such as *Toxoplasma, Leishmania* and *Plasmodium* (Saeij et al. 2005; Franke-Fayard et al. 2006; Dellacasa-Lindberg et al. 2007; Hutchens and Luker 2007). Very recently BLI has also been applied to the pathogenic yeast *Candida albicans* to investigate virulence in vaginal mucosal and systemic infections (Doyle et al. 2006a).

BLI on Gram-negative and positive bacteria can easily be performed by introducing the the *luxCDABE* operon from *Photorhabdus luminescens* or *Xenorhabdus luminescence* (Hutchens and Luker 2007) or the *luxABCDE* operon with optimised binding sites for Gram-positive bacterial ribosomes (Francis et al. 2000), respectively. The use of BLI in infection models revealed new insights on the dissemination of pathogens and was, furthermore, suitable to study the effect of antibiotic treatment on clearance of infection (Francis et al. 2001; Hardy et al. 2004).

Since the bacterial system is not suitable for eucaryotes and viruses, the luciferase coding region from the firefly *Photinus pyralis* or from the sea pansy *Renilla reniformis* is cloned under the control of a promoter, which is active during disease development. Light emission is enforced by intraperitoneal or intraveinous injection of D-luciferin or coelenterazine, depending on the source of luciferase, whereby D-luciferin emitts light at > 560 nm enabling the detection from deeper tissues than possible with coelenterazine (light emission at 480 and 490 nm). In addition, D-luciferin is non-toxic for mammalian cells and highly water soluble, which makes the substrate available at all body sites. The only additional prerequisite for light production is the availability of oxygen, which is involved in the light producing luciferase reaction (Hutchens and Luker 2007).

The first and, up to now, only example for *in vivo* monitoring of pathogenesis caused by a fungal species by BLI in a murine infection model is that described for *C. albicans.* Constitutive luciferase gene expression was enforced by use of the *ENO1* promoter (Doyle et al. 2006b). Vaginal mucosal infections were successfully monitored and the use of the antifungal drug miconazole revealed the disapearance of bioluminescence, which coincide with a reduced fungal load (Doyle et al. 2006a). In a systemic infection strong bioluminescence was detected from the kidneys, which were already shown to be the main targeted organs in systemic *C. albicans* infections (Papadimitriou and Ashman 1986). However, luminescence was not detected from other organs, probably due to a low colonisation rate or because of a reduced uptake of luciferin in the yeast hyphal form, which turned out as one of the major drawbacks in this study (Doyle et al. 2006a).

In the present invention, the Inventors have generated bioluminescent filamentous fungus strains by introducing a luciferase gene controled by a promoter allowing expression of said luciferase in a filamentous fungus host cell. Resulting strains were suitable to study the effectiveness of antifungals *in vitro* since bioluminescence correlated well with fungal growth. Moreover, bioluminescence imaging from infected mice enabled to follow disease development *in vivo* since the fungal burden directly correlated with the light emission from infected organs. Intravenous application of conidia led to a rapid appearance of luminescence from the heart region but manifestation occured especially in the liver and kidneys. In contrast, no dissemination was observed after intranasal conidia application. Therefore, bioluminescence imaging not only provides a suitable tool to study disease development, localisation of fungal growth and determination of the burden but may also provide a brilliant tool to study the effectiveness of new antifungals *in vivo.*

Existing technologies to monitor fungal infections were limited to the observation of snapshots of the infection process by killing infected mice at various time points, which did not allow monitoring of disease progression in single animals. In addition, discovering the sites of infection was a laborious work and was limited to the organs selected for histopathology and might have been not sufficient, because some infected organs might have been missed. The present invention has allowed the discovery of new sites of infection which were not known before.

The technique can be used to monitor the establishment of infection mediated by filamentous fungal strains such as *Aspergillus* strains, and can also be applied to infection of different transgenic mouse strains. The system is suitable for monitoring the effectiveness of drugs in clearance of mycelium and is therefore independent of large cohorts of animals to determine survival curves. In addition, a time dependence of clearance of mycelium from infected sites can be monitored.

Consequently, in a first aspect, the present invention relates to a nucleic acid comprising a gene encoding a luciferase which is under the control of a promoter for expression, advantageously constitutive expression, of said luciferase in a filamentous fungal host cell.

In the present invention, the nucleic acid comprises any promoter which allows expression, preferably constitutive expression, of the luciferase in a filamentous fungal host cell. The term "constitutive expression" means that the expression is carried out in the cell without particular regulation.

The promoter may be of any origin, provided that it allows expression, preferably constitutive expression, of the luciferase in a filamentous fungal host cell. Preferably, the promoter is from a filamentous fungal gene, such as a gene of genus *Aspergillus (A.), Fusarium* or *Rhizopus, Mucor, Rhizomucor, Scedosporium* or *Phaeohyphomycoses,* said list being not limitative. More preferably, the promoter from a filamentous fungal gene is from the same filamentous fungal genus as the genus of the filamentous fungal host cell.

More preferably, the promoter is from an *Aspergillus* gene. Also more preferably, the promoter is from an *A. fumigatus* gene, from an *A. nidulans* gene, from an *A. terreus* gene or from an *A. oryzae* gene. More preferably, the promoter is from an *A. fumigatus* gene.

Preferably, the promoter allowing expression of the luciferase in a filamentous fungal host cell is not a promoter from a *Candida albicans* gene.

Advantageously, the promoter is a promoter of a gene selected from the group consisting of genes involved both in gluconeogenesis and glycosis, and genes involved in the pentose pathway. Such genes are well known by the biochemist. More advantageously, the gene promoter involved both in gluconeogenesis and glycosis is the phosphoglucose isomerase promoter, the aldolase promoter, the triosephosphate isomerase promoter, the glyceraldehyde-3-phosphate dehydrogenase (gpdA) promoter (PgpdA), the phosphoglycerate kinase promoter, the phosphoglycerate mutase promoter, the enolase promoter.

Preferably, the gene promoter involved in the pentose phosphate pathway is the glucose-6-phosphate dehydrogenase promoter, the 6-phosphogluconolactonase promoter, the phosphogluconate dehydrogenase promoter, the ribulose-5-phosphate isomerase promoter or the ribulose-5-phosphate epimerase promoter.

More preferably, the gene promoter is involved both in gluconeogenesis and glycosis. More preferably, the gene promoter involved both in gluconeogenesis and glycosis is the glyceraldehyde-3-phosphate dehydrogenase (gpdA) promoter (PgpdA).

Most preferably, the promoter for expression of the luciferase in a filamentous fungal host cell is the glyceraldehyde-3-phosphate dehydrogenase (gpdA) promoter (PgpdA) from *A. fumigatus* whose sequence is selected among anyone of the following sequences:
a) the sequence SEQ ID N°1,
b) a sequence having at least 70 %, preferably 72 %, 75 %, 77 %, 80 %, 82 %, 85 %, 87 %, 90 %, 91 %, 92 %, 93 %, 94 %, 95 %, 96 %, 97 %, 98 %, or 99 % of identity with SEQ ID N°1 which retains the activity of the glyceraldehyde-3-phosphate dehydrogenase promoter from *Aspergillus fumigatus,* and
c) a sequence complementary to anyone of the sequences defined in a) and b).

The terms nucleic acid, nucleotidic acid, polynucleotide, etc are used indifferently herein to design a nucleic acid sequence. In the same way, the expressions protein, polypeptide and peptide are used indifferently herein to design an amino acid sequence or their derivatives or analogues containing an amino acid sequence.

By percentage of identity between two amino acid or nucleic acid sequences in the present invention, it is meant a percentage of identical amino acid residues or nucleotides between the two sequences to compare, obtained after the best alignment ; this percentage is purely statistical, and the differences between the two sequences are randomly distributed and all along their length. The best alignment or optimal alignment is the alignment corresponding to the highest percentage of identity between the two sequences to compare, which is calculated such as herein after. The sequence comparisons between two nucleic acid or amino acid sequences are usually performed by comparing these sequences after their optimal alignment, said comparison being performed for one segment or for one "comparison window", to identify and compare local regions of sequence similarity. The optimal alignment of sequences for the comparison can be performed manually or by means of the algorithm of local homology of Smith and Waterman (1981) (Ad. App. Math. 2:482), by means of the algorithm of local homology of Neddleman and Wunsch (1970) (J. Mol. Biol. 48:443), by means of the similarity research method of Pearson and Lipman (1988) (Proc. Natl. Acad. Sci. USA 85:2444), by means of computer softwares using these algorithms (GAP, BESTFIT, FASTA and TFASTA in the Wisconsin Genetics Software Package, Genetics Computer Group, 575 Science Dr., Madison, WI).

The percentage of identity between two nucleic acid or amino acid sequences is determined by comparing these two aligned sequences in an optimal manner with a "comparison window" in which the region of the nucleic acid or amino acid sequence to compare may comprise additions or deletions with regard to the sequence of reference for an optimal alignment between these two sequences. The percentage of identity is calculated by determining the number of positions for which the nucleotide or the amino acid residue is identical between the two sequences, by dividing this number of identical positions by the total number of positions in the "comparison window" and by multiplying the result obtained by 100, to obtain the percentage of identity between these two sequences.

The term "luciferase" refers in the present invention to an enzyme that reacts with its substrate luciferin in the presence of oxygen and adenosine triphosphate (ATP), said reaction resulting in photon production, i.e. bioluminescence. Bioluminescence is a naturally occurring phenomenon which is commonly used as a reporter system. The expressions "luciferase", "bioluminescent reporter protein" and "reporter protein" refer in the present invention to the same enzyme. Also in the present invention, the term "luciferin" refers to the substrate of luciferase which, when it reacts with luciferin in the presence of oxygen and ATP, results in photon production process, i.e. bioluminescence. The expressions "luciferin", "substrate luciferin" and "luminescent substrate" refer herein to the substrate of the enzyme luciferase. Also in the present invention, the luciferase is encoded by the nucleic acid, and the filamentous fungal host cell having been transformed with said nucleic acid will emit photons, and thus light, upon addition of the luminescent substrate and in the presence of oxygen and ATP.

The luciferin may be of any origin, such as for example firefly luciferin or coelenterazine luciferin. The luciferin that may be used in the present invention is well known by the skilled person.

In the present invention, any appropriate device can be used for measuring the quantity of the emitted bioluminescence. As an example, the *in vitro* measurement of the quantity of emitted bioluminescence can be performed with the luciferase assay described by BD Biosciences (Erembodegem, Belgium) or with a modification of said luciferase in a manner similar to that disclosed at point I.4 of the Examples below. Alternatively, for *in vitro* or *in vivo* measurement, the device which may be used is the IVIS 100 system (Xenogen Corporation, Alameda, USA) and the images are acquired according to the manufacturer's instructions. Analysis and acquisition can be performed using Living Image software version 2.6 and 3 (Xenogen/Caliper). It is thus possible to acquire images from the exterior of mice, from internal organs during dissection or from cultures in plates. Quantification of photons per second emitted by each organ can be performed by defining regions of interest corresponding to the respective organ of interest (Examples, point I.9 infra). In a general manner, the devices for measuring the quantity of the emitted bioluminescence are well known from the skilled person, who will be able to adapt them if necessary.

Any gene encoding a luciferase can be used in the present invention. Advantageously, the luciferase gene is from bacteria, such as the luciferase gene of terrestrial *Photorhabdus luminescens* or of marine *Vibrio harveyi,* or from eukaryotes. The bacterial or eukaryotic luciferase genes are well known and are commonly used by the skilled person. More advantageously, the luciferase gene is from eukaryotes. Preferably, the eukaryotic luciferase gene is from beetles, from the sea pansy *Renilla,* such as *Renilla reniformis,* from the jellyfish *Aequorea,* from the small crustacean *Vargula,* or from the single-cell aquatic organism *Gonyaulax.* More preferably, the eukaryotic luciferase gene is from beetles, such as fireflies or click beetles. More preferably, the eukaryotic luciferase gene is from the firefly *Photinus,* such as *Photinus pyralis.*

Most preferably, the eukaryotic luciferase gene is from *Photinus pyralis* and has a sequence selected among anyone of the following sequences:
a) the sequence SEQ ID N°2,
b) a sequence having at least 70 %, preferably 72 %, 75 %, 77 %, 80 %, 82 %, 85 %, 87 %, 90 %, 91 %, 92 %, 93 %, 94 %, 95 %, 96 %, 97 %, 98 %, or 99 % of identity with SEQ ID N°2 encoding a protein which retains the activity of the firefly *Photinus pyralis* luciferase, and
c) a sequence complementary to anyone of the sequences defined in a) and b).

In the present invention, the expression "filamentous fungal host cell" means a host cell of a microscopic fungus wherein the hypha (long, branching, filamentous cell) is the base element and a hyphea network constitutes the mycelium. The filamentous fungal host cell according to the present invention is either pathogenic in a healthy living organism, or opportunistic, i.e. biologically saprophyte but pathogenic in an immunosuppressed living organism. In the present invention, the healthy or immunosuppressed living organism that may be infected by the filamentous fungal host cell is a human or animal being, or a plant.

Examples of filamentous fungal host cells that infect the healthy or immunosuppressed, human or animal being are fungal host cells of the genus *Aspergillus, Fusarium,* such as *Fusarium solani, Rhizopus,* such as *Rhizopus oryzae, Mucor, Rhizomucor, Scedosporium* or *Phaeohyphomycoses,* this list being not limitative.

Examples of filamentous fungal host cells that infect the plant are *Botrytis cinerea* (pathogenic) and *Neurospora crassa* (opportunistic).

As precised above, the promoter from a filamentous fungal gene is preferably from the same filamentous fungal genus as the genus of the filamentous fungal host cell.

Preferably, the filamentous fungal host cell is an *Aspergillus* host cell. More preferably, the *Aspergillus* host cell is an *A. fumigatus* host cell, an *A. nidulans* host cell, an *A. terreus* host cell. Most preferably, the *Aspergillus* host cell is an *A. fumigatus* host cell.

In a further preferred embodiment, the nucleic acid according to the present invention comprises anyone of the following sequences:
a) the sequence SEQ ID N°3 corresponding to the firefly *Photinus pyralis* luciferase which is under the control of the glyceraldehyde-3-phosphate dehydrogenase promoter from *Aspergillus fumigatus,*
b) a sequence having at least 70 %, preferably 72 %, 75 %, 77 %, 80 %, 82 %, 85 %, 87 %, 90 %, 91 %, 92 %, 93 %, 94 %, 95 %, 96 %, 97 %, 98 %, or 99 % of identity with SEQ ID N°3 which retains the activity of the glyceraldehyde-3-phosphate dehydrogenase promoter from *Aspergillus fumigatus* and which encodes a protein which retains the activity of the firefly *Photinus pyralis* luciferase, and
c) a sequence complementary to anyone of the sequences defined in a) and b).

Advantageously, the nucleic acid according to the present invention further comprises a gene encoding a selectable marker of the transformed filamentous fungal host cell.

It is known that, depending on the expression vector and transfection technique used, only a small fraction of host cells may integrate the foreign DNA into their genome. In order to identify and select these integrants, a gene that encodes a selectable marker (e.g., resistance to antibiotics, auxotrophy, etc...) is generally introduced into the host cells. Any gene encoding an appropriate selectable marker can be used in the present invention, provided that it allows the selection of the transformed filamentous fungal host cell. Preferably, the gene encoding a selectable marker is from a filamentous fungus, such as a fungus of the genus *Aspergillus, Fusarium,* such as *Fusarium solani,* or *Rhizopus,* such as *Rhizopus oryzae, Mucor, Rhizomucor, Scedosporium* or *Phaeohyphomycoses,* this list being not limitative. More preferably, the gene encoding a selectable marker is from *Aspergillus.* More preferably, the gene encoding a selectable marker is from *A. fumigatus, A. nidulans, A. terreus* or from *A. oryzae.* Most preferably, the gene encoding a selectable marker is from *A. oryzae.* Examples of genes encoding a selectable marker are the hygromycin resistance gene, the phleomycin resistance gene or the pyrithiamine resistance gene, this list being not limitative. The hygromycin resistance gene has been used in a variety of fungi. Examples include *Histoplasma capsulatum* (Woods et al., 1998), *Aspergillus fumigatus* and different *Fusarium* strains such as *Fusarium solani* (Crowhurst et al, 1992). An example for use of phleomycin resistance as a selection marker for transformations of *Aspergillus flavus* is given in He et al., 2007 or for *Trichoderma* species (Cardoza et al., 2006).

Advantageously, the gene encoding a selectable marker for selection of the transformed filamentous fungal host cell is the pyrithiamine resistance gene. Also advantageously, the gene encoding a selectable marker for selection of the transformed *Aspergillus* host cell is the pyrithiamine resistance gene. More advantageously, the pyrithiamine resistance gene is from a filamentous fungus, such as a fungus of the genus *Aspergillus, Fusarium,* such as *Fusarium solani,* or *Rhizopus,* such as *Rhizopus oryzae, Mucor, Rhizomucor, Scedosporium* or *Phaeohyphomycoses,* this list being not limitative. More advantageously, the pyrithiamine resistance gene is from *Aspergillus.* More advantageously, the pyrithiamine resistance gene is from *A. fumigatus, A. nidulans, A. terreus* or from *A. oryzae.* Most advantageously, the pyrithiamine resistance gene is from *A. oryzae* and has a sequence selected among anyone of the following sequences:
a) the sequence SEQ ID N°4,
b) a sequence having at least 70 %, preferably 72 %, 75 %, 77 %, 80 %, 82 %, 85 %, 87 %, 90 %, 91 %, 92 %, 93 %, 94 %, 95 %, 96 %, 97 %, 98 %, or 99 % of identity with SEQ ID N°4 encoding a protein which retains the resistance activity to the pyrithiamine,
c) a sequence complementary to anyone of the sequences defined in a) and b).

In a most preferred embodiment, the nucleic acid according to the present invention comprises anyone of the following sequences:
a) the sequence SEQ ID N° 5 corresponding to the firefly *Photinus pyralis* luciferase which is under the control of the glyceraldehyde-3-phosphate dehydrogenase promoter from *Aspergillus fumigatus,* and to the pyrithiamine resistance gene from *Aspergillus oryzae* upstream to the promoter,
b) a sequence having at least 70 %, preferably 72 %, 75 %, 77 %, 80 %, 82 %, 85 %, 87 %, 90 %, 91 %, 92 %, 93 %, 94 %, 95 %, 96 %, 97 %, 98 %, or 99 % of identity with SEQ ID N°5 which retains the activity of the glyceraldehyde-3-phosphate dehydrogenase promoter from *Aspergillus fumigatus,* which encodes a protein which retains the activity of the firefly *Photinus pyralis* luciferase, and which further encodes a protein which retains the resistance activity to the pyrithiamine, and
c) a sequence complementary to anyone of the sequences defined in a) and b).

The nucleic acid according to the present invention is obtained using conventional techniques. Methods of DNA engineering are sufficiently described in the literature, and are well known by the man skilled in the art, who will know how to use them in the most convenient manner in the process of the invention (see Sambrook et al. (1989), Molecular Cloning: A Laboratory Manual, Cold Spring Harbor Laboratory Press, Cold Spring Harbor, N.Y.; by Ausubel et al. (1994), eds Current Protocols in Molecular Biology, Current Protocols Press; and by Berger and Kimmel (1987), Methods in Enzymology: Guide to Molecular Cloning Techniques, Vol. 152, Academic Press, Inc., San Diego, Calif. the disclosures of which are hereby incorporated by reference).

In a second aspect, the present invention relates to a vector comprising the nucleic acid of the present invention, as described above.

By the term vector or plasmid in the sense of the present invention, it is meant a DNA or RNA, circular or linear molecule, which is indifferently in the form of a single or double strand. A vector according the invention is preferably an expression vector. It may be a vector of bacterial or viral origin. It should of course be understood that not all vectors will function equally well in the present invention. However, one of skill in the art may make a selection among these vectors without undue experimentation.

In a third aspect, the present invention relates to a transformed filamentous fungal host cell comprising the nucleic acid of the present invention or the vector of the present invention, as defined above.

In the present invention, "transformed filamentous fungal host cell" means that the filamentous fungal host cell expresses the luciferase.

Nucleic acids or vectors of the invention can be introduced into filamentous fungal host cells growing in culture in vitro by conventional transfection techniques (e.g., calcium phosphate precipitation, DEAE-dextran transfection, electroporation etc.). Such transfection techniques can be found in Sambrook, et al. (Molecular Cloning: A Laboratory Manual. 3rd, ed., Cold Spring Harbor Laboratory, Cold Spring Harbor Laboratory Press, Cold Spring Harbor, N. Y. , 2001), and other laboratory manuals. Preferably, the standard protoplast transformation as described in Balance and Turner (1985) is used for obtaining the transformed *Aspergillus* host cells. The point 1.5 below of the Examples discloses in detail the operating process using this protoplast transformation. Said operating process may be adapted if necessary. Moreover, appropriate fungus culture media to be used for cultivating transformed *Aspergillus* host cells are well known from the man skilled in the art, such as Sabouraud agar or 2 % malt extract media.

Advantageously, the transformed filamentous fungal host cell is a transformed *Aspergillus* host cell. More advantageously, the transformed *Aspergillus* host cell is a transformed *A. fumigatus* host cell, a transformed *A. nidulans* host cell, a transformed *A. terreus* host cell or a transformed *A. oryzae* host cell. More preferably, the transformed *Aspergillus* host cell is a transformed *A. fumigatus* host cell. Most preferably, the transformed filamentous fungal host cell is a transformed *Aspergillus fumigatus* host cell which has been deposited at the CNCM (Collection Nationale de Cultures de Microorganismes, Institut Pasteur, 25, rue du Docteur Roux, F-75725 PARIS CEDEX 15) on February 27, 2008 and has the accession number CNCM I-3928. The strain CNCM I-3928 deposited on February 27, 2008 at the CNCM is also named herein indifferently *"Aspergillus fumigatus* C3 luminescent strain" or "AfC3 strain".

In a further aspect, the present invention relates to an animal model which has been infected with the transformed filamentous fungal host cell of the present invention, said animal model being advantageously an immunosuppressed animal model. The term "animal model" as used herein refers to a non-human mammal including, without limitation, laboratory animals such as rodents, advantageously a mouse model.
The term "immunosuppressed" or "immunocompromised" is used indifferently herein and means that the animal model has reduced immune defense mechanisms. Said immunosuppression is obtained using any appropriate technique, and the immunosuppressive techniques are well known by the skilled person, such as by treating the animal model with immunosuppressive agents, by irradiation or by inoculating antibodies in order to deplete granulocytes. Indeed, reduced immune defense mechanisms on the host side are generally required to allow fungal outgrowth. The immunosuppressive agents may be for example a cytotoxin, such as cyclophosphamide, or a corticosteroid, such as cortisone acetate. Non limiting examples of immunosuppressive regimen are:
- with cortisone acetate: In brief, mice are immunosuppressed with two single doses of 25 mg cortisone acetate (Sigma) injected intraperitoneally 3 days before and immediately prior to infection with conidia (day 0);
- granulocyte depletion: the monoclonal antibody MAb RB6 (gift) specific for the IgG2B antibody (100µg) diluted in sterile phosphate-buffered saline has been inoculated intraperitoneally; or
- cyclophosphamide treatment: 200mg/kg of cyclophosphamide are injected intraperitoneally 4 and 1 day before as well as 2 days after infection.

The animal model may be infected either by intravenous, intranasal, oral or any other convenient administration of the transformed filamentous fungal host cell of the present invention.

The invention also relates to a method for determining the virulence of a filamentous fungal host cell according to the present invention, which comprises the following steps:
a) the transformation of the filamentous fungal host cell with the nucleic acid according to the present invention or with the vector according to the present invention,
b) the culture of the transformed filamentous fungal host cell in order to obtain a transformed filamentous fungal host cell population,
c) the addition to the culture of luciferin which is capable to induce bioluminescence upon reaction under appropriate conditions with the luciferase expressed by the transformed filamentous fungal host cell population,
d) the *in vitro* detection of the emitted bioluminescence, whereby said detection is made advantageously immediately after the step (c) of addition of luciferin, and
e) the determination that the filamentous fungal host cell population is *in vivo* virulent, for example in an infected animal being such as mice, when bioluminescence is detected.
   "Appropriate conditions" at step (c) means that conditions for obtaining bioluminescence are fulfilled, and include the presence of oxygen and ATP

The present invention allows determining the pathogenesis caused by filamentous fungal host cells, such as mutant strains which show hypo- or hyper-virulence. Indeed, in the case where conidia, which are the reproductive cells of fungi, are not able to germinate, there will be no significant increase in the bioluminescent signal.

The present invention also relates to the use of the transformed filamentous fungal host cell according to the invention for studying a filamentous fungal infection, advantageously for studying specific host immune response mechanisms in response to a filamentous fungal infection. For example, when the transformed filamentous fungal host cell is a transformed *Aspergillus* host cell, the invention relates to the use of the transformed *Aspergillus* host cell for studying an aspergillus infection.

The present invention further relates to the use of the animal model according to the invention for the monitoring of the time-dependent progression of a filamentous fungal infection, advantageously for studying specific host immune response mechanisms in response to a filamentous fungal infection.

The bioluminescence of the transformed filamentous fungal host cells according to the invention is switched on in all growth states, which allows the study of both *in vitro* and *in vivo* conditions. The system is thus suitable for monitoring the time-dependent progression of fungal infections, either after inhalation, swallowing or intravenous application of the transformed filamentous fungal host cells.

The present invention also relates to the use of a culture of transformed filamentous fungal host cells of the invention or of an animal model of the invention for the *in vitro* determination of the antifungal activity of a compound.

The expressions "compound having antifungal activity", "antifungal compound", "antifungal drug", etc are used indifferently herein and refer to any compound whose efficacy in the treatment of a filamentous fungal infection can be evaluated with the host cells, animal models and methods of the present invention.

The present invention further relates to a method for determining the antifungal activity of a compound, which comprises the following steps:
a) the transformation of a filamentous fungal host cell with the nucleic acid according to the invention or with the vector according to the invention,
b) the culture of the transformed filamentous fungal host cell in order to obtain a transformed filamentous fungal host cell population,
c) the addition to the culture of the compound whose antifungal activity is to be determined,
d) the addition to the culture of luciferin which is capable to induce bioluminescence upon reaction under appropriate conditions with the luciferase expressed by the transformed filamentous fungal host cell population,
e) the quantitative measurement of the emitted bioluminescence, whereby said quantitative measurement is made advantageously immediately after the step (d) of addition of luciferin, and
f) the comparison of the measured quantity of emitted bioluminescence obtained in step (e) with that obtained for the same filamentous fungal host cell whithout addition of the compound whose antifungal activity is to be determined, and
g) the determination that the compound has antifungal activity when the measured quantity of emitted bioluminescence obtained in step (e) is inferior to that obtained for the same filamentous fungal host cell whithout addition of the compound.
   "Appropriate conditions" at step (d) means that conditions for obtaining bioluminescence are fulfilled, and include the presence of oxygen and ATP

The present invention further relates to a method for determining the antifungal activity of a compound which comprises the following steps:
a) the administration of the compound whose antifungal activity is to be determined, to an animal model according to the invention,
b) the administration of luciferin which is capable to induce bioluminescence upon reaction under appropriate conditions with the luciferase expressed by the transformed filamentous fungal host cell, to the animal model,
c) the quantitative measurement of the emitted bioluminescence in at least one organ of the animal model, whereby said quantitative measurement is made advantageously immediately after the step (b) of administration of luciferin, and
d) the comparison of the measured quantity of emitted bioluminescence obtained in step (c) with that obtained in the organ for the same filamentous fungal host cell whithout addition of the compound whose antifungal activity is to be determined, and
e) the determination that the compound has antifungal activity when the measured quantity of emitted bioluminescence obtained in step (c) is inferior to that obtained for the same filamentous fungal host cell whithout addition of the compound.
   "Appropriate conditions" at step (b) means that conditions for obtaining bioluminescence are fulfilled, and include the presence of oxygen and ATP

In an alternative embodiment of the methods for determining the antifungal activity of a compound as described above, the obtained measured quantity of emitted bioluminescence can be compared with that obtained for the same filamentous fungal host cell which has been treated with another compound whose antifungal activity is known.

Preferably, in the case of an animal model according to the invention which has been infected with a transformed *Aspergillus* host cell, the method for determining the antifungal activity of a compound by means of said animal model comprises an additional step (f) of monitoring the health status of the animal model, such as monitoring a variation of the body weight and/or the mobility of the animal model. Indeed, in cases where lungs are strongly infected by *Aspergillus,* luminescence drops down after having reached a peak value of light emission. This is due to extremely poor clinical state of the animals avoiding the transportation of the substrate luciferin, and the necrotic area in the lung resulting in the limitation of the oxygen. Therefore, monitoring the health status of the animal helps to discriminate between reduction of the fungal burden by antifungal drugs and the reduction of the signal due to the illness of the animals. It is also possible to circumvent the limitations of the system by using a serial dilution of conidia, which enables a longer period of survival of infected animals and reduces the number of necrotic areas, which in turn enables a better oxygenation of the infected tissues. As a consequence, the emitted bioluminescence will also remain measurable for a prolonged time.

In a further embodiment, the methods for determining the antifungal activity of a compound according to the invention further allow to determine the effective dose of the antifungal compound required to control fungal growth.

The present invention also relates to the glyceraldehyde-3-phosphate dehydrogenase promoter (PgpdA) from *Aspergillus fumigatus* as such, whose sequence is selected among anyone of the following sequences:
a) the sequence SEQ ID N°1,
b) a sequence having at least 70 %, preferably 72 %, 75 %, 77 %, 80 %, 82 %, 85 %, 87 %, 90 %, 91 %, 92 %, 93 %, 94 %, 95 %, 96 %, 97 %, 98 %, or 99 % of identity with SEQ ID N°1 which retains the activity of the glyceraldehyde-3-phosphate dehydrogenase promoter from *Aspergillus fumigatus,* and
c) a sequence complementary to anyone of the sequences defined in a) and b).

The present invention also relates to the use of a nucleic acid comprising a gene encoding a luciferase which is under the control of a promoter as described above for expression of said luciferase in a filamentous fungal host cell.

The examples and figures below illustrate the invention but do not limit its scope in any way.

### FIGURES

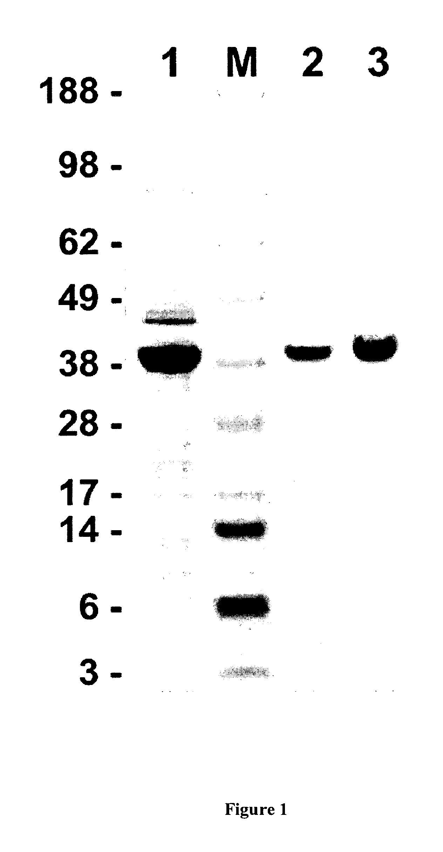

**Figure 1****: SPS-PAGE showing the purification of recombinant glyceraldehyde-3-phosphate dehydrogenase (GpdA) from A. *fumigatus.*** Lane 1: 25 µg of *E. coli* crude extract overproducing *A. fumigatus* GpdA; lane 2: 2.4 µg purified GpdA; lane 3: 3.5 µg purified GpdA; lane M: molecular mass standard. Masses of the marker (in kDa) are shown on the left side.

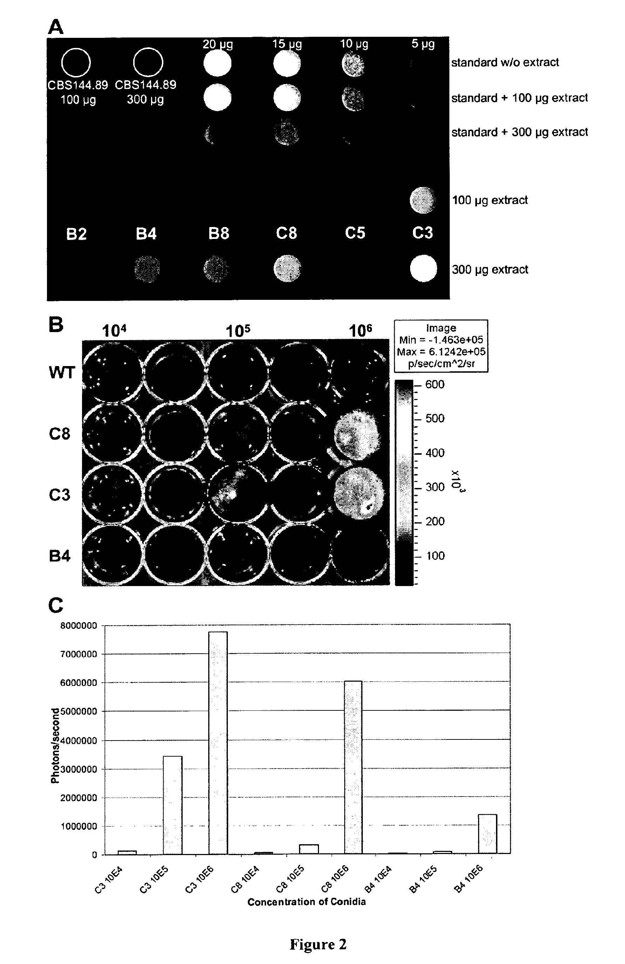

**Figure 2****: *In vitro* and *in vivo* bioluminescence of selected *A. fumigatus* strains producing the firefly luciferase. A:** Digital picture showing light emission from crude extracts of the transformants B2, B4, B8, C8, C5 and C3 after growth on glucose. The parental wild-type strain CBS 144.89 and recombinant luciferase (in the range of 5 to 20 µg) with and without the addition of wild-type crude extract served as controls. Addition of crude extract slightly quenched the light emission from the luciferase control. **B:** *In vitro* determination of light emission by use of the IVIS 100 system. Different conidia concentrations (1 × 10⁴, 1 × 10⁵, 1 × 10⁶) of the wild-type strain and the three transformants C8, C3 and B4 were inoculated in RPMI medium and incubated for 8 h at 37°C. Light emission was induced by the addition of D-luciferin to the medium C: Quantification of light emission of wells shown in (B) by the Living Image software version 3.0.

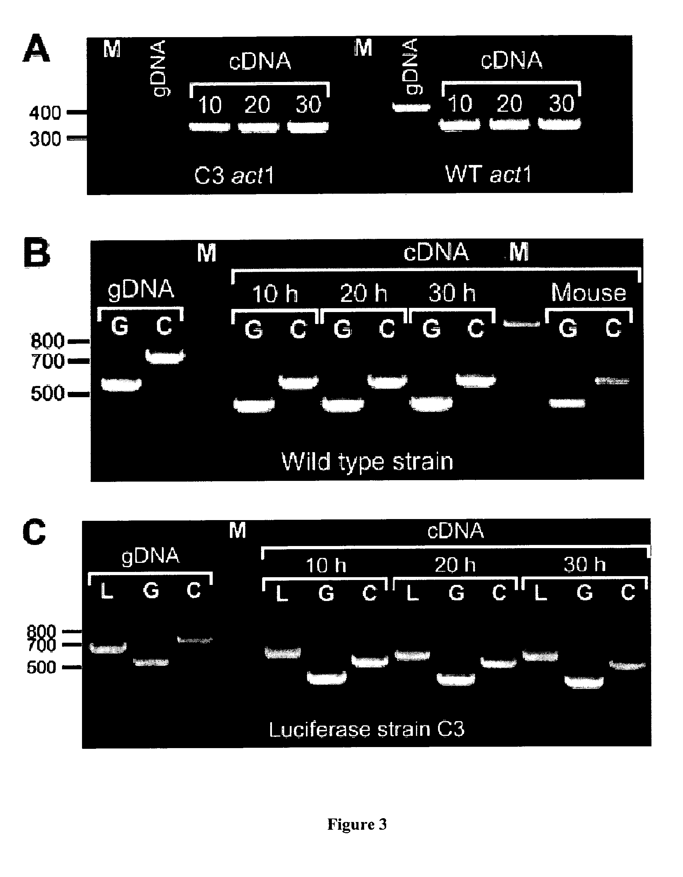

Figure 3: Determination of the relative abundance of transcript levels of ***gpdA, luc* and *citA (citrate synthase gene).*** cDNA from glucose/peptone grown mycelium, incubated for 10, 20 and 30 h as well as cDNA from an infected mouse lung were studied. **A:** Standardisation of the cDNA's against the actin gene *act1.* Numbers above the cDNA amplification products denote the incubation times of the respective strains. Genomic DNA (gDNA) served as controls to visualise the size shift in the cDNA amplification. Fragment size for gDNA: 432 bp; cDNA: 354 bp. **B:** Determination of *gpdA* (G) and *citA* (C) transcript levels on standardised amounts of cDNA from the wild type and on cDNA from an infected mouse lung. Genomic DNA (gDNA) served as a control. Transcript sizes: gDNA for *gpdA*: 564 bp; cDNA for *gpdA:* 451 bp; gDNA for *citA*: 754 bp; cDNA for *citA:* 575 bp. Boxed values denote the ratios of band intensities as calculated from trace quantity determinations. The *gpdA* transcript levels stay constant, whereas those of *citA* slightly decline in later growth phases. The *gpdA* transcript from the infected mouse lung is much stronger pronounced than that of *citA,* resembling a late growth phase of the fungus in severe infection. **C**: Same as in B, but the luciferase producing strain C3 was studied and the *luc* gene (L) was included. Fragment sizes for both, gDNA and cDNA are identical (688 bp), since the *luc* gene does not contain an intron. Boxed values denote the ratios of band intensities as calculated from trace quantity determinations. Transcript levels for *luc* and *gpdA* stay constant, whereas that of *citA* steadily declines.

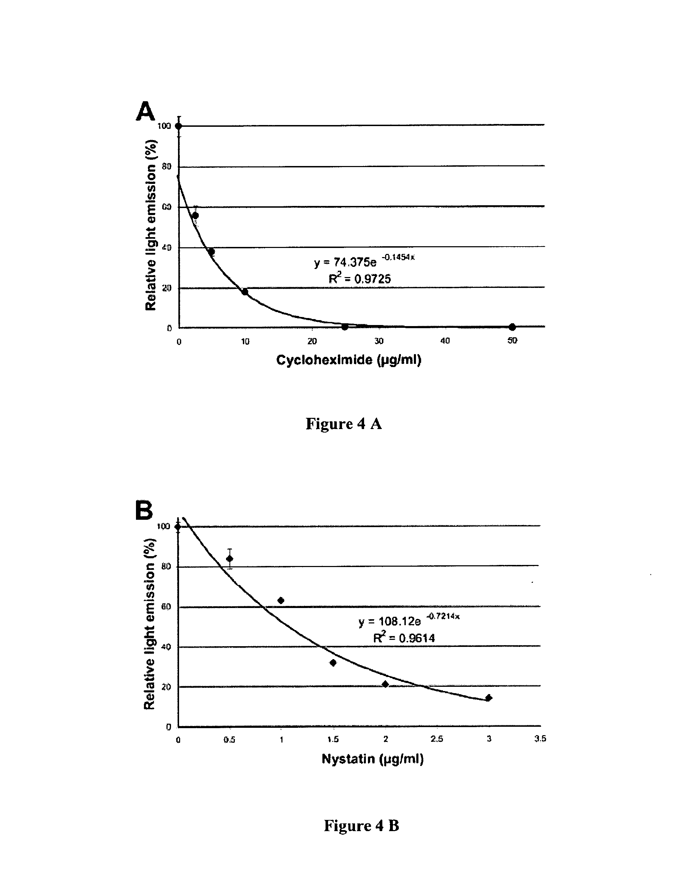

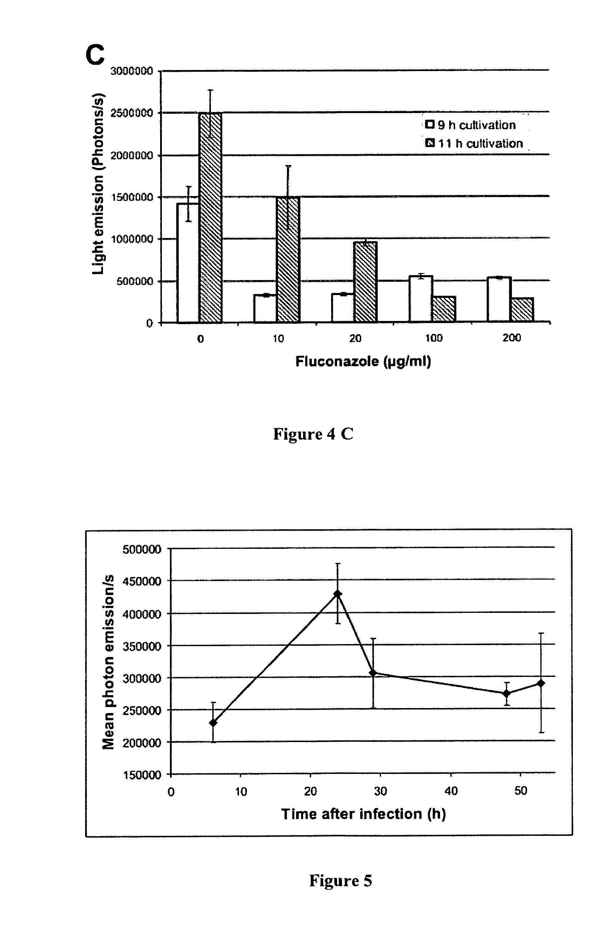

**Figure 4****: Graphical analysis of drug efficiency by luminescence detection.** **Fig 4A****:** Light emission from wells containing 50,000 conidia of *A. fumigatus* strain C3 and addition of different amounts of cycloheximide. Measurement was performed in a microplate reader after 15 h of incubation at 37°C. Standard deviations were calculated from 3 independent wells for each concentration. A fitted trend line with the corresponding formula and the correlation coefficient is given. **Fig 4B****:** Same as in (A) but Nystatin was used as the antifungal drug. **Fig 4C****:** Determination of light emission from strain C3 by the IVIS 100 system after growth in the presence of different concentrations of fluconazole. Light emission was detected after 9 and 11 h of incubation in RPMI complete medium at 37°C. Average values from two independent wells are given and the mean deviation is shown by error bars.
**Figure 5****: Time-lapse study of luminescence emission from BALB/cJ mice intranasally infected with 2 × 10⁶ conidia of strain C3.** The graph shows the mean light emission from the chests of mice as determined by data analysis with the Living Image software version 3.0. Standard deviations are given by error bars.
   All mice display luminescence from their lungs 23 h after infection, when D-luciferin was injected intraperitoneally (data not shown). Luminescence stayed visible until death of animals but declined steadily. The lungs from mice No. 1, 3 and 4, which were removed post mortem, displayed strong luminescence after direct injection of D-luciferin. Light emission from live animals was recorded for 5 min, whereas the exposure time for organs *ex vivo* was set to 1 min.

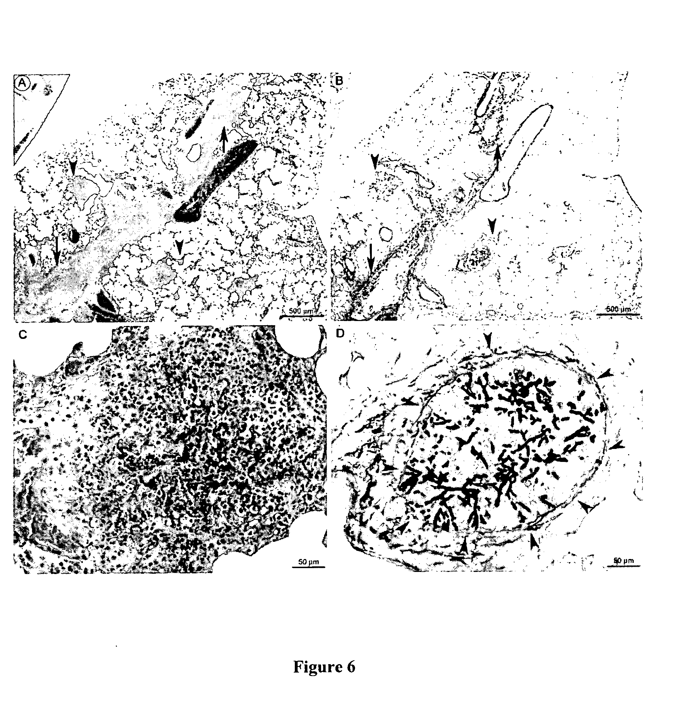

**Figure 6****: Lung histopathology of BALB/cJ mice intranasally infected with the bioluminescent *A. fumigatus* strain C3. A and B:** Multifocal inflammatory lesion, generally centred on bronchi and bronchioles (arrows), and rarely extending to alveoli and blood vessels (veins and arteries) (arrowheads), characterised by **C**: an infiltration of karyorrhectic neutrophils (suppuration) and erythrocytes (haemorrhage), a destruction of the bronchial/bronchiolar overlying epithelium (necrosis), and **D:** the presence of intralesional fungal hyphae. These hyphae generally did not cross the bronchiolar wall (arrowheads). A, C: haematoxylin and eosin staining; B, D: Grocott's methenamine silver staining.

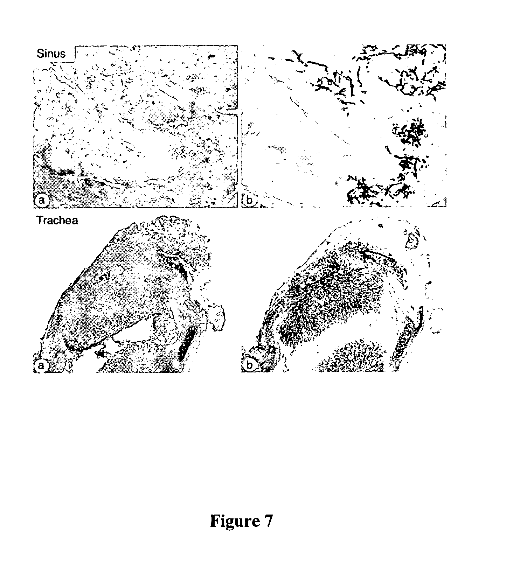

**Figure 7****: Histopathological analysis of the sinuses revealed for mouse (3) an inflammatory lesion (multifocal to coalescing suppurative sinusitis) with sinus bone and cartilage remodelling and very numerous intralesional fungal hyphae**

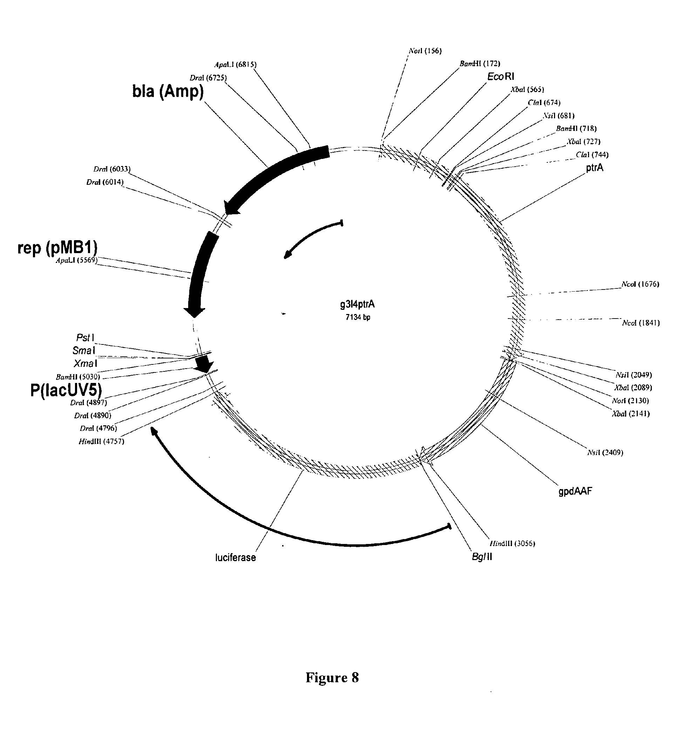

**Figure 8****: Map of the vector PgpdA::luc/pJET/ptrA" issued from the pJET1/blunt cloning vector.**

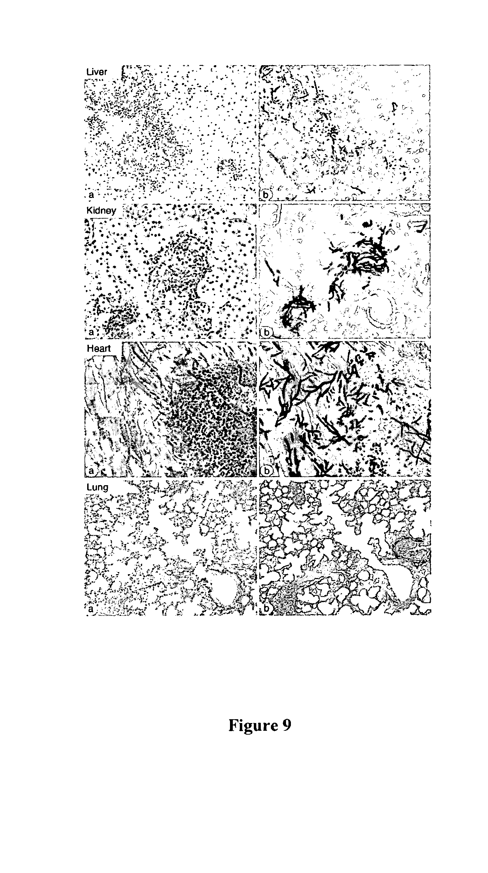

**Figure 9****: Histopathology of the organs again revealed a very good correlation between the luminescence signal and the fungal burden within these tissues.**
**Figures 10** **and** **11****: Survival immunosupression**

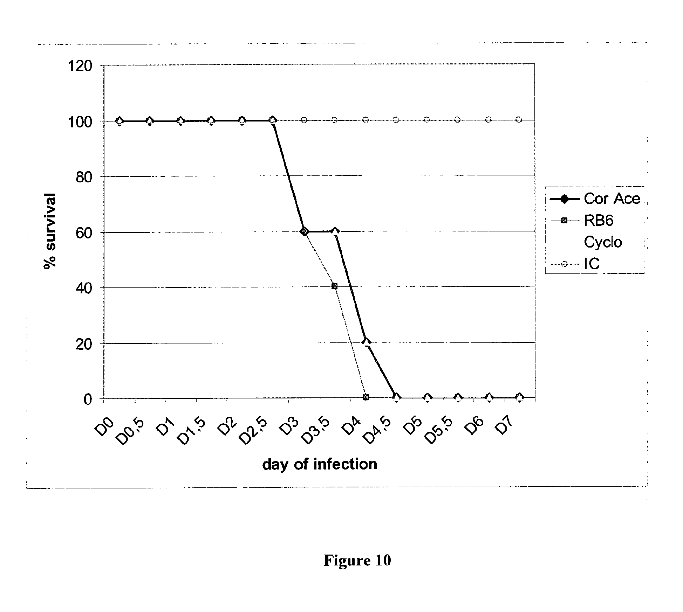

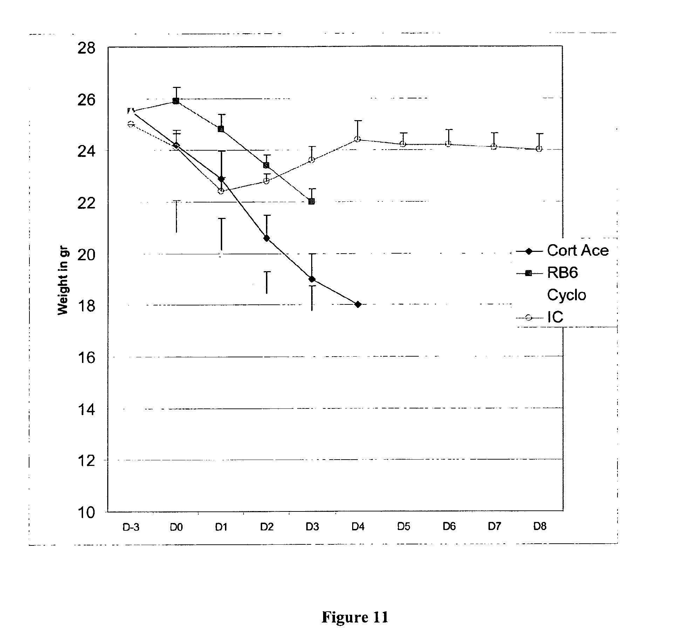

   **Fig 10** shows the survival curve of immunosuppressed compared to immunocompetent BALB/c mice infected intranasally with 2 × 10⁶ conidia. Under the different immunosuppression regimen, all the animals die between 4 and 4.5 days post infection compared to 100% survival for immunocompetent animals. The susceptibility to infection of each group was also followed up by the loss in weight **(****Fig11****).** After 1D (D = day) infection all the animals wether immunosuppressed or not displayed a slight decrease in their weights. Cyclophosphamide ("cyclo") treated mice show the highest 32 % of loss with a moribund shape compared to 30 and 28% of loss observed for animals immunosuppressed with cortisone acetate after infection with 2 × 10⁵ or 2 × 10⁶ conidia. RB6 treated animal show only 14% weight-loss. Unlike the immunosuppressed animals, immunocompetent mice display a weight increase after 1day infection. "Cor Ace" means cortisone acetate and "IC" means immunocompetent.

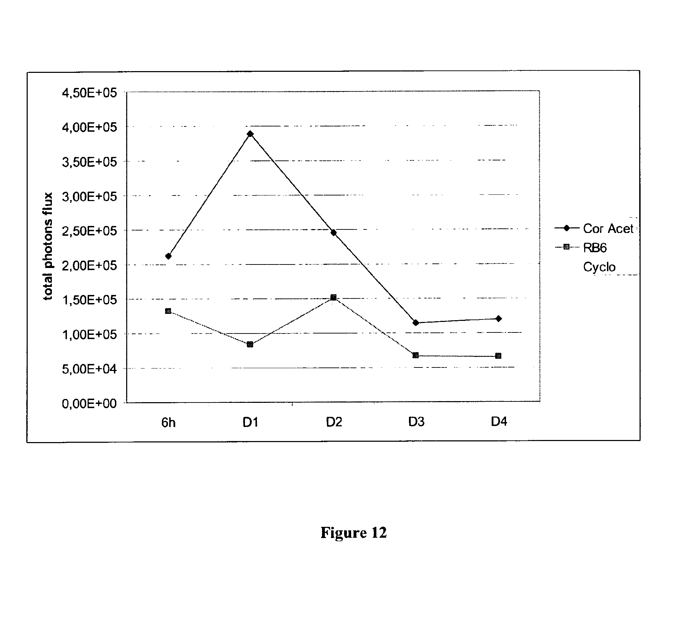

**Figure 12****: Quantification of the luminescence measured after different** times of **infection** Each point represents the average of total photon flux measured from a defined chest region of interest from each individual animal. After 24h infection the highest luminescence was visible in the chest area of the cortisone acetate treated mice. The peak of luminescence was observed after 2D (2 days) of infection in the RB-6 treated animals. The cyclophosphamide treated animals show a delayed luminescence starting to increase from D2 with the highest value at D4 (Images of the highest luminescence detected after 1, 2 or 4 days from animals treated with cortisone acetate, RB-6 antibodies and cyclophosphamide not shown).

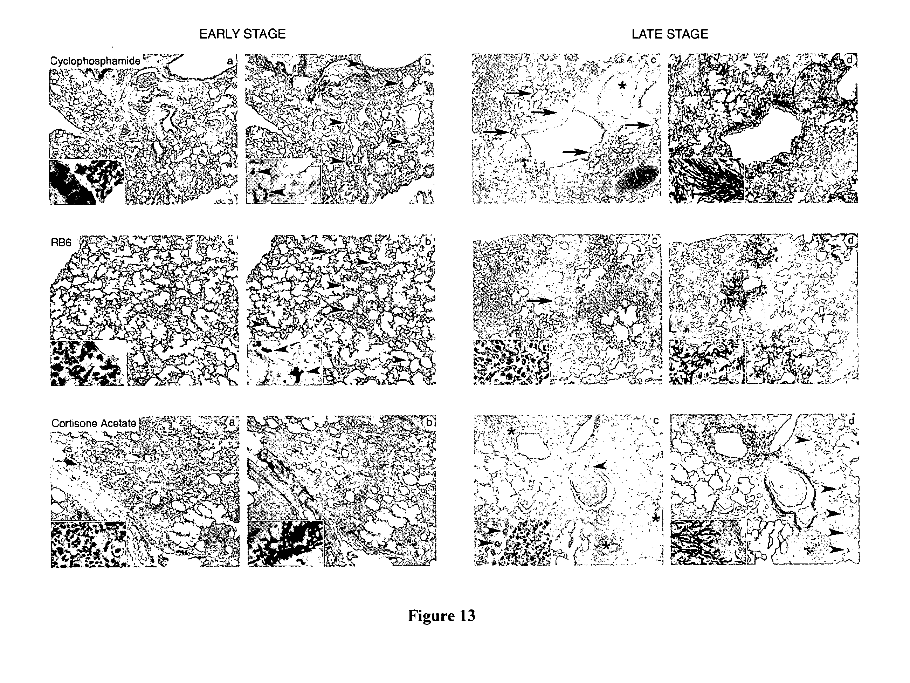

**Figure 13****: Hemalun or Grocott's-stained lung sections from the different groups of animals sacrificed after 1 day infection (early stage) or died after 3 and 4.5 days (late stage).** In the cyclophosphamide treated animals within the early stage, only small groups of neutrophils could be observed within bronchiolar spaces (inset; star) without any significant histological lesion or modification of the overlying epithelium (a). Non-germinating and very rare early-germinating conidia were multifocally detected in bronchiolar and alveolar spaces using the Grocott's staining (arrowheads) (b). In contrast, in the late stage of pulmonary infection, a multifocal to coalescing lesion was observed. It was characterized by a total lack of inflammatory response and a destruction of the broncho-alveolar structure (inset) because of a fungal severe and extensive infiltration of the pulmonary parenchyma and vascular walls and spaces, leading to thrombosis (star) and infarcts.

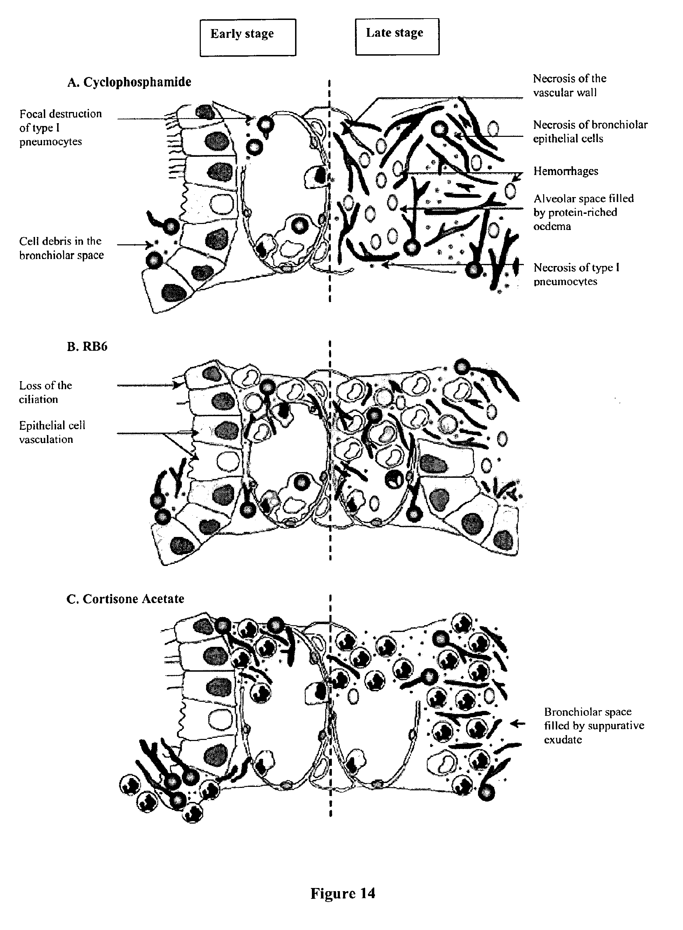

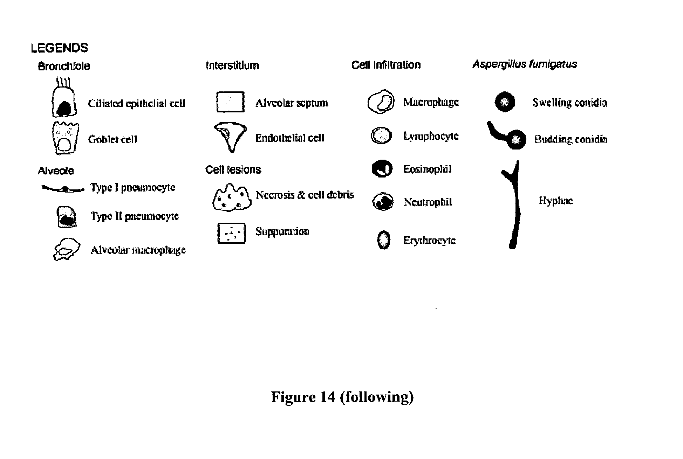

**Figure 14****: A**t an early stage (24 hours post-infection), cyclophosphamide and RB6 immunosuppressive conditions were characterised by the presence of only swollen and early germinating conidia. These observations coincided with the data obtained by the BLI analysis. Luminescence from the lungs was indeed at a low level for the cyclophosphamide and RB6 treatments (1.5 x 10⁵ and 1.0 x 10⁵ photons/mm² respectively). Inflammatory cells detected in these two conditions were macrophages only (cyclophosphamide) or associated with lymphocytes and plasma cells (RB6). No neutrophil was observed.

In contrast, in the cortisone acetate condition, even if alveolar macrophages that sometimes had phagocytosed conidia could also be noted, the main infiltrating cell type was neutrophils. They were observed in bronchial/bronchiolar spaces, as well as in interalveolar septae and alveolar spaces. In addition to swollen, germinating conidia could be observed resulting from non phagocytosed conidia. It is well described in the literature that a cortisone acetate treatment could lead to anomalies in monocyte counts and in macrophage phagocytosis ability and oxygen-dependant microbial killing mechanisms (REFERENCES). Collectively, these data would suggest that in the early phase of the pulmonary colonisation by *A. fumigatus,* the alveolar macrophage plays a really significant role in the clearance of conidia.
In the late stage, unlike what we described for the early stage, cyclophosphamide and RB6 treatments ended with a strong growth of fungi, associated with a high infiltration of the pulmonary parenchyma (mostly in the case of the cyclophosphamide treatment; lung bioluminescence: 2.55 x 10⁵ photons/mm²). Hyphae had indeed been observed in the bronchial/bronchiolar/alveolar spaces, in interalveolar septae, as well as infiltrating vascular walls. In the same way as what we found in the early stage, no neutrophil was detected in any of these two conditions.
Cortisone acetate treatment was characterised by an almost similar density of fungi, but with low infiltration ability. Fungi were indeed limited to bronchial/bronchiolar spaces, and rare fungi were observed multifocally in alveolar spaces. In contrast to cyclophosphamide and RB6 conditions, several infiltrating neutrophils could be observed in the lesions. Collectively, these data would suggest that once the conidia germinate, neutrophils are very important in circling the fungi and limiting their infiltration in the lung parenchyma.

### EXAMPLES

### I - Experimental procedures

Table 1 summarised all the alignments used in the present invention

**Table 1: Oligonucleotides used in this study.**

| **Name** | **Target gene** | **Sequence** **(5' → 3')** | **Restriction site** | **Function** | **Sequence number** |
|---|---|---|---|---|---|
| PgpdAAf Not_up | *gpdA* | GCG GCC GCA GAT TCT AGA AGT CCT G | *Not*I | Amplification *gpdA* promoter | SEQ ID N°9 |
| PgpdAAfB glATG | *gpdA* | AGA TCT CAT TGT GTA GAT TCG TCT GGT AC | *Bgl*II | Amplification *gpdA* promoter | SEQ ID N°10 |
| Anchored Oligo(dT)₂₀ | mRNA | NVT TTT TTT TTT TTT TTT TTT T | --- | Generation of cDNA | SEQ ID N°23 |
| BglAfGPD cDNA_up | *gpdA* | AGA TCT GCC ACT CCC AAG GTT GG | *Bgl*II | Amplification cDNA *gpdA* | SEQ ID N°13 |
| HindAfGP DcDNA_d o | *gpdA* | AAG CTT ACT GGG AAT CGA CCT TGG | *Hind*III | Amplification cDNA *gpdA* | SEQ ID N°14 |
| LucBgl_up | *luc* | AGA TCT GAC GCC AAA AAC ATA AAG AAA GG | *Bgl*II | Amplification *luc* | SEQ ID N°6 |
| LucHind_ down | *luc* | AAG CTT ACA CGG CGA TCT TTC CGC | *Hind*III | Amplification *luc* | SEQ ID N°7 |
| LucMi_up | *luc* | GAT GTC CAC CTC GAT ATG TG | --- | Control primer | SEQ ID N°8 |
| Not_ptrA_ up_for | *ptrA* | GCG GCC GCT TGA TTA CGG GAT CCC ATT GG | *Not*I | Amplification *ptrA* | SEQ ID N°11 |
| Not_ptrA_ dow_rev | *ptrA* | GCG GCC GCG TAT TAT ACT GTC TTT CTT GTT ACA | *Not*I | Amplification *ptrA* | SEQ ID N°12 |
| cDNAgpd AAf_up | *gpdA* | CGT CGA TGC TCC CTC CAA C | --- | RT-PCR *gpdA* | SEQ ID N°15 |
| cDNAgpd AAf_do | *gpdA* | GGA ATC GAC CTT GGC GAT G | --- | RT-PCR *gpdA* | SEQ ID N°16 |
| lucProbe_ up | *luc* | GAT GGA ACC GCT GGA GAG C | --- | *luc* probe / RT-PCR *luc* | SEQ ID N°17 |
| lucProbe_ | *luc* | CCA AAA CCG | --- | *luc* probe / | SEQ ID |
| mi_rev | | TGA TGG AAT GGA AC | | RT-PCR *luc* | N°18 |
| AfCitAcod e_up | *citA* | GCA AGG TCA TCG GCG AGG | --- | RT-PCR *citA* | SEQ ID N°19 |
| AfCitAcod e_do | *citA* | GTT GTC ACC GTA GCC GAG C | --- | RT-PCR *citA* | SEQ ID N°20 |
| act_Afum_ for_II | *act1* | GCT TTG CTA CGT CGC TCT CG | --- | RT-PCR *act1* | SEQ ID N°21 |
| act_Afum_ rev_II | *act1* | GGA GGA GCA ATG ATC TTG ACC | --- | RT-PCR *act1* | SEQ ID N°22 |

### I.1 Cloning of the Photinus pyralis luciferase gene

The firefly luciferase gene, optimised for eucaryotic gene expression, was amplified from plasmid pSP-luc⁺ (Promega) by PCR using a proofreading polymerase (Accuzyme, Bioline). The 5' oligonucleotide LucBgl_up (SEQ ID N°6: 5' - AGA TCT GAC GCC AAA AAC ATA AAG AAA GG - 3') contained a *Bgl*II restriction site, which led to the modification of the ATG start-codon and that of the second codon of the luciferase gene. The 3' oligonucleotide LucHind_down (SEQ ID N°7: 5' - AAG CTT ACA CGG CGA TCT TTC CGC - 3') contained a *Hind*III restriction site (restriction sites shown in bold). The resulting 1656 bp PCR fragment was gel purified and cloned into the PCR blunt end cloning vector pJET1/blunt (GeneJET PCR cloning kit; MBI Fermentas). *E. coli* DH5α cells were transformed with the ligation mixture and positive clones were pre-selected by colony PCR with oligonucleotides LucBgl_up and LucMi_up (SEQ ID N°8: 5' - GAT GTC CAC CTC GAT ATG TG - 3'). Positive clones were transferred to liquid medium and plasmid DNA was purified by use of the E.Z.N.A. plasmid miniprep kit II (PeqLab). Orientation of the insert was checked by independent *Hind*III and *Bgl*II restriction. *Hind*III digested plasmids, in which a 270 bp fragment of the vector sequence was removed, were purified from the gel, self-ligated and transferred to DH5α cells. By this procedure the additional *Bgl*II restriction site was removed from the cloning vector, which led to the plasmid pJETluc-Bgl. Plasmid DNA was isolated as described above and used for downstream cloning procedures.

### I.2 Cloning of the gpdA promoter from A. fumigatus

The promoter sequence of the glyceraldehyde-3-phosphate dehydrogenase (*PgpdA*) from *A. fumigatus* was selected for constitutive expression of the luciferase gene. For that purpose a BLAST search on fungal genomes was performed at the NCBI BLAST server (http://www.ncbi.nlm.nih.gov/BLAST/) using the protein sequence of the *Aspergillus nidulans* glyceraldehyde-3-phosphate dehydrogenase (Accession P20445) as a template. By that means the putative *A. fumigatus* glyceraldehyde-3-phosphate dehydrogenase, which displayed 83% identity to the *A. nidulans* enzyme, was identified (locus tag: Afu5g01970). The CADRE database (http://www.aspergillus.org.uk/indexhome.htm?secure/sequence_info/index.php∼main) was used to identify the genomic upstream region and a 970 bp fragment, including the ATG start codon was taken as the respective promoter region. The promoter was amplified from genomic DNA of the *A. fumigatus* wild-type strain CBS 144.89 by a proofreading polymerase as described above. Oligonucleotides used were PgpdAAfNot_up (SEQ ID N°9: 5' - GCG GCC GCA GAT TCT AGA AGT CCT G - 3'; *Not*I restriction site in bold) and PgpdAAfBglATG (SEQ ID N°10: 5' - AGA TCT *CAT* TGT GTA GAT TCG TCT GGT AC - 3'; *Bgl*II restriction site in bold and reverse complement sequence of the ATG start codon in italics). The fragment was gel purified, subcloned into the pJET1/blunt cloning vector, transferred to DH5α cells and resulting clones were pre-screened by colony PCR. Plasmid DNA was isolated from positive clones and subjected to a *Not*I and *Bgl*II double restriction to release the promoter region from the cloning vector.

### I.3 Construction of the final transformation plasmid:

The plasmid pJETluc-Bgl was restricted with *Bgl*II and *Not*I to enable the in frame fusion of the luciferase coding region with the glyceraldehyde-3-phosphate dehydrogenase promoter. After gel purification of the restricted vector the promoter fragment from above was ligated to the vector leading to the plasmid PgpdA::luc/pJET. The ligation products were transferred to *E. coli* DH5α cells and positive clones were selected by colony PCR with the oligonucleotides PgpdAAfNot_up and LucMi_up. Plasmid DNA was purified and restricted with *Not*I for subsequent ligation of the pyrithiamine resistance gene (*ptrA*) from *Aspergillus oryzae.* For that purpose the *ptrA* gene was amplified from pSK275 (kindly provided by S. Krappmann, Georg-August-Universität Goettingen, Germany) with the oligonucleotides Not_ptrA_up_for (SEQ ID N°11: 5' - GCG GCC GCT TGA TTA CGG GAT CCC ATT GG - 3') and Not_ptrA_dow_rev (SEQ ID N°12: 5' - GCG GCC GCG TAT TAT ACT GTC TTT CTT GTT ACA - *3'; Not*I restriction sites shown in bold) and ligated into the pJET1/blunt cloning vector leading to plasmid NotptrA/pJET. The *ptrA* gene was released by *Not*I restriction and subcloned into the previously *Not*I restricted vector PgpdA::luc/pJET. Positive clones were identified by colony PCR on the *ptrA* gene and plasmid DNA (PgpdA::luc/pJET/ptrA) was purified for transformation of *A. fumigatus.*

### I.4 Cloning of the luciferase gene for recombinant production in E. coli and luciferase assay:

The luciferase gene from plasmid pJETluc-Bgl was excised by *Bgl*II and *Hind*III double restriction and subcloned into a previously *Bam*HI and *Hind*III digested pET43.1bHis₆ histidine-tag expression vector (Novagen). The ligation mixture was transferred to *E. coli* DH5α cells. Plasmid DNA was purified and transferred to *E. coli* BL21 (DH3) Rosetta2 cells (Novagen). Four independent clones were picked and inoculated each in 15 ml of auto-induction medium (Overnight Express Instant TB Medium, Novagen). After 20 h of aerobic incubation at 30°C the cells had reached an optical density of OD₅₅₀ of 22 - 25. An aliquot of each culture, to give a final density of OD₅₅₀ of 10 in 1 ml of extraction buffer (30 ml Tricine, 10 mM DTT, 0.5 mM EDTA, 5 mM MgCl₂; pH 7.8), was removed and cells were disrupted by sonication. After centrifugation both, the cell free supemantant and the pellet, solubilised in 8 M urea, were subjected to SDS-PAGE analysis using 4-12% Bis-Tris gradient gels from Invitrogen and the MES/SDS running buffer system (Invitrogen). Coomassie staining revealed that a significant proportion of the produced luciferase was present in inclusion bodies. To check for luciferase activity in the soluble fraction, the cell free supernatant was used for detection of luciferase activity. The assay was performed by a modification of the luciferase assay described by BD Biosciences (Erembodegem, Belgium). The double concentrated assay buffer contained: 30 mM Tricin, 15 mM MgCl₂, 10 mM DTT, 5 mM ATP and 1 mM free Coenzyme A; pH 7.8. 100 µl of each extract were transferred to white 96 well plates and mixed with 90 µl of assay buffer. The reaction was started by addition of 10 µl D-luciferin (15 mM stock) and the assay was read after 5 min in a multiplate reader (FLUOstar OPTIMA, BMG LABTECH) with a signal gain of 1000. All samples emitted values of 28 000 to 32 000 relative response units, which proved the functionality of the luciferase gene. To use this luciferase as a standard for subsequent measurements the four cultures were pooled (60 ml in total) and cell pellets were resuspended in buffer A (20 mM HEPES buffer pH 7.5 with 150 mM NaCl) and opened by sonication. The cell free extract was loaded on a Ni-chelate column (14 ml bed volume). The column was washed first with buffer A (5 column volumes) subsequently with buffer B (buffer A + 30 mM imidazol; 4 column volumes) and luciferase was finaly eluted in buffer C (buffer A + 200 mM imidazol). The eluted fractions contained pure luciferase and were desalted and concentrated *via* centrifugal filter devices (Millipore; 30 kDa cutoff). The concentrated samples were diluted in extraction buffer + 50% (final concentration) of glycerol for storage at - 20°C. Protein determination revealed a total yield of 1.5 mg purified luciferase from the 60 ml of culture volume. The purified recombinant luciferase was used for standardisation of light emission of fungal extracts.

### I.5 Transformation of A. fumigatus and initial screening procedure:

Two independently constructed plasmids of PgpdA::luc/pJET/ptrA (for construction see above) were used for transformation of the *A. fumigatus* wild-type strain CBS144.89. The transformation was performed by the standard protoplast transformation as described elswhere (Ballance and Turner 1985). In brief, conidia were inoculated in 200 ml of *Aspergillus* minimal medium (AMM) with 50 mM glucose as the carbon source and incubated at 220 rpm on a rotary shaker at 37°C for 18 h. After washing the mycelium was incubated in glucanex containing buffer (400 mg in 20 mL) for 2 h at 30°C. Protoplasts were filtered off, washed, counted and resuspended in transformation buffer. For each transformation 6 µg of unrestricted plasmid DNA was used. The transformation mix was transferred to Top-agar containing AMM with glucose, 2% agar and the addition of 0.6 M KCl and 1 mM pyrithiamine and poured on Bottom plates with the same composition. Plates were incubated at 37°C for 5 days and conidia of colonies were transferred several times to pyrithiamine containing media to yield pure transformed clones. 16 clones were randomly selected and some conidia were scraped from the colonies and transferred to 200 µl of AMM glucose medium in white 96 well plates. After incubation for 14 h at 37°C 90 µl of luciferase assay buffer and 10 µl of luciferin were added. Wells were observed with the naked eye in the dark for green light emission. The six strongest light-emitting clones were used for further analysis.

### I.6 Cloning, recombinant production and purification of GpdA from A. fumigatus

To confirm that the predicted *gpdA* promoter indeed belongs to the glyceraldehyde-3-phosphate dehydrogenase coding region, RNA was isolated from peptone grown mycelium and a total cDNA was prepared by use of anchored oligo(dT)₂₀ primers as described (Ibrahim-Granet et al. 2008). Specific amplification of the *gpdA* gene by the Phusion Hot start polymerase (Finnzymes) was performed in a total reaction volume of 10 µl using the Speed cycler technology (http://www.analytik-jena.de/frontend/index.php?itid=2296&). Oligonucleotides used were BglAfGPDcDNA_up (SEQ ID N°13: 5'- *AGA TCT* GCC ACT CCC AAG GTT GG -3') containing an *Bgl*II restriction site (italic) and the oligonucleotide HindAfGPDcDNA_do (SEQ ID N°14: 5'- *AAG CTT* ACT GGG AAT CGA CCT TGG -3'), which contained a *HindIII* restriction site (italic). The resulting PCR fragment was cloned into the pJet cloning vector as described above and sequenced from both strands to determine the correct open reading frame. After *Bgl*II/*Hind*III double digestion the *gpdA* gene was subcloned into a pET43.1bHis₆ vector as described for cloning of the luciferase gene. After transfer of the plasmid to *E. coli* BL21 (DE3) Rosetta2 cells protein production was induced in Overnight Express Instant TB Medium by growing the cells for 22 h at 28°C. Cells were resuspended in 50 mM Tricine buffer pH 8.0 containing 150 mM NaCl and 20 mM imidazole and were disrupted by sonication. Glyceraldehyde-3-phosphate dehydrogenase activity was determined virtually as described previously with slight modifications (Brenneman and Volk 1959). A typical assay in a final volume of 1 mL contained: 50 mM Tricine pH 8.0; 10 mM NAD; 4 mM fructose-1,6-bisposphate; 2 mM dithiothreitol; 20 mM potassium arsenate (pH 8.0); 5 mM potassium phosphate (pH 8.0); 0.8 U aldolase (from rabbit muscle) and GpdA containing enzyme preparations. Generation of NADH was monitored at 340 nm and specific activity was calculated as µmol of NADH oxidation × min⁻¹ × mg⁻¹ of protein. In order to proof that maximum activity was only achieved in the presence of arsenate, it was substituted by 45 mM potassium phosphate in the assay mixture. For purification the soluble fraction of the crude extract was loaded on a Ni-chelate column previously equilibrated with Tricine/NaCl buffer pH 8.0. After a stringency wash in the presence of 30 mM imidazole pure protein was eluted from the column as determined by SDS polyacrylamide gel electrophoresis by use of a NuPage 4-12% Bis-Tris gradient gel (Invitrogen). The protein band was excised and subjected to a tryptic digestion with sequencing grade modified trypsin as described by the manufacturer (Promega). Peptides were mixed with α-cyano-4-hydroxycinnamic acid and analysed by MALDI TOF. Additionally, some peptides were selected for sequencing by MALDI TOF MS-MS analysis.

### Activity determination and expression analyses of gpdA, citA and luc

In order to confirm that the *gpdA* promoter is constitutively active during growth on various carbon sources, the inventors cultivated the *A. fumigatus* wild type strain CBS144.89 and the luciferase producing strain C3 on liquid media with different nutrient compositions. Cells were grown for 20 h on RPMI complete cell culture medium or on minimal media containing glucose, ethanol or a mixture of glucose and peptone. For the latter composition we were not only interested in the expression of *gpdA* at a single time point, but also in the expression profile over time. Therefore, mycelia grown on glucose/peptone were harvested after 10, 20 and 30 h. The inventors first determined the specific activities of GpdA, CitA (citrate synthase gene) and luciferase as depicted in table 2. Comparison of the activity of GpdA from the 20 h cultures revealed that activity was weakest on RPMI medium, but rather constant on ethanol and glucose and slightly elevated on glucose/peptone medium. The biomass formed on RPMI medium only made up approximately one third of that obtained from glucose or glucose/peptone after 20 h and determination of the residual glucose concentration revealed a total consumption of this substrate. This indicates that, due to the high amount of conidia used for inoculation (2 × 10⁶/ml), mycelium was faced with glucose starvation. Nevertheless, GpdA, luciferase and citrate synthase activity were still highly abundant. Interestingly, GpdA activity increased over time, when the 10, 20 and 30 h glucose/peptone cultures from both strains were compared (glucose was still present after 30 h). This was assumed to be either due to an increased gene expression in later growth states or to a high stability of GpdA, which might accumulate during growth. In contrast, luciferase activity only varied slightly over time and correlated quite well with the GpdA activity determined from the fixed 20 h values. Citrate synthase is also known to be a constitutively produced enzyme of *A. fumigatus,* but is additionally induced on ethanol, because it also serves as part of the glyoxylate cycle within the glyoxysomes. However, in contrast to GpdA, citrate synthase activity slightly decreased in later growth phases, indicating that the production level of this enzyme is not constant.

To correlate the observed enzymatic activities with the gene expression, the inventors selected the mycelia from the different time points of glucose/peptone grown mycelia and isolated RNA, which was subsequently transcribed into cDNA. Additionally, cDNA was prepared from a mouse lung, which was infected with the *A. fumigatus* wild type and was sacrificed five days after infection because of severe physical distress and strong signs of dyspnoea.

The inventors assumed that the actin gene would serve as a suitable marker to compare gene expression, since all cultures were still increasing in their biomass, making the continued synthesis of actin necessary. Therefore, the cDNA levels were normalised to the transcript level of the *act1* gene (figure 3A), whereby this standardisation was not possible for the cDNA isolated from the mouse lung, because even 250 ng template cDNA only yielded a very faint band, which was hardly detectable and most likely due to the small proportion of cDNA of fungal origin in this sample.

Standardised cDNA was used to amplify the *gpdA* and *citA* transcript from the wild type (approximately 5 ng template) and from the infected mouse lung (250 ng). From the luciferase producing strain C3 the *luc, gpdA* and *citA* transcripts were investigated (figure 3B,C). In order to calculate the ratio of transcript levels we calculated the trace quantities of each band (area under the intensity profile curve) and correlated the resulting data (ratios are given as boxed numbers in figure 3). This analysis revealed that indeed the *citA* transcript level decreased in older mycelium whereas the *gpdA* expression stayed constant and, furthermore, that the ratio between the *luc* and the *gpdA* transcripts also stayed constant. Quantification of the transcript levels from the infected mouse lung showed a high proportion of *gpdA* transcripts, whereas that of *citA* revealed a much weaker signal, which correlates with a mycelium in a later growth phase.

These data clearly showed that: (i) *gpdA* is a constitutively expressed gene, although some variations in dependence on the carbon source may occur; (ii) *luc* gene expression correlates well with *gpdA* expression; (iii) expression of *gpdA* and *luc* is constant over time on a given carbon source; (iv) *gpdA* is still expressed when mice are suffering from the fungal infection. Therefore we conclude that the *gpdA* promoter is indeed a suitable tool for constitutive luciferase production on a given nutrient.

**Table 2:**

| Determination of GpdA, CitA and luciferase activities from the *A. fumigatus* wild-type strain (WT) and the luciferase producing strain C3. No light emission was observed for the wild type (n.d. = not detectable). RRU/mg denote relative response units of light emission per mg of total protein. All light emission values were within the range of a standard curve recorded with purified luciferase. All data derived from triplicate measurements and the standard deviation is given. CS = citrate synthase activity. | | | | | | |
|---|---|---|---|---|---|---|
| **Carbon source / growth time** | **GpdA WT** **(mU/mg)** | **GpdA C3** **(mU/mg)** | **CS WT** **(mU/mg)** | **CS C3** **(mU/mg)** | **Luciferase WT** **(RRU/mg)** | **Luciferase C3** **(RRU/mg)** |
| RPMI/2 0 h | 587 ± 30 | 608 ± 32 | 307 ± 8 | 311 ± 5 | n.d. | 14906 ± 750 |
| EtOH/2 0h | 986 ± 150 | 906 ± 55 | 958 ± 34 | 785 ± 28 | n.d. | 31520 ± 1938 |
| Gluc/20 h | 990 ± 58 | 1019 ± 3 | 343 ± 17 | 364 ± 12 | n.d. | 32207 ± 1032 |
| Gluc+P ep/10 h | 849 ± 41 | 689 ± 9 | 350 ± 20 | 305 ± 7 | n.d. | 74610 ±2816 |
| Gluc+P ep/20 h | 1339 ± 103 | 1169 ± 24 | 242 ± 10 | 221 ± 5 | n.d. | 51010 ± 2643 |
| Gluc+P ep/30 h | 1671 ± 96 | 1316 ± 20 | 203 ± 8 | 143 ± 4 | n.d. | 52437 ± 127 |

### I.7 Strain culturing and mouse infection

The bioluminescent *A. fumigatus* strain C3 was selected and subcultured on 2% malt extract agar slants for 8 days at room temperature. Conidia were harvested by scrapping conidia from the slant culture with 2 mL of phosphate buffered saline supplemented with 0.1 % Tween 20 (PBST). The suspension was filtered through a 40 µm cell strainer (Falcon) to separate conidia from contaminating mycelium.

Male outbred Swiss OF1 or BALB/cJ mice (20-24 g, 6 weeks old) were supplied by the Centre d'Elevage R. Janvier, Le Genest Saint-Isle, France and used at about 8 weeks of age for infection. Mice were fed normal mouse chow and water *ad libitum* and were reared and housed under standard conditions with air filtration. Mice were cared for in accordance with Pasteur Institute guidelines in compliance with the European animal welfare regulation. Intranasal infection and immunosuppression were performed as previously described (Schöbel et al. 2007). In brief, mice were immunosuppressed with two single doses of 25 mg cortisone acetate (Sigma) injected intraperitoneally 3 days before and immediately prior to infection with conidia (day 0). Before infection, mice were anesthetised with an intramuscular injection of 0.1 mL of a solution containing 10 µg/ml ketamine (Merial, France) and 2 µg/ml xylazine (Bayer, Leverkusen, Germany) per mouse. For the inhalation model two different concentrations of conidia of 2 × 10⁶ and 7 × 10⁷ conidia (unless indicated otherwise) in 25 µl of PBS 0.1% Tween 20 were applied to the nares of the mice using an automatic pipetting device. For intravenous infection 2 × 10⁶ conidia in 100 µl of PBS 0.1% Tween 20 were injected into the tail vein of immunosuppressed mice. As a control for monitoring the clearance of conidia in immunocompetent animals, mice were infected intranasaly with 2 × 10⁶ conidia and observed daily by IVIS measurements. In all experiments mice were kept for a maximum of 15 days post infection. Weight loss of mice was determined daily.

### I.8 Antifungal drug efficiency testing by luciferase producing A. fumigatus strains

The luciferase producing strains C3 and C8 were chosen for testing the growth inhibitory effect of the model antibiotic cycloheximide (Baliga et al. 1969). White 96 well plates (Nunc) were used for inoculation and subsequent measurement of luciferase activity. Before plates were inoculated a sterilisation for 1 h with short wavelenght UV-light was performed. Each well contained 50 000 conidia of the respective strain and cycloheximide ranging in a concentration between 0 µg/mL and 50 µg/mL. *Aspergillus* minimal medium (for composition see at: http://www.fgsc.net/methods/anidmed.html) with 50 mM glucose and 0.5% (w/v) peptone served as growth medium and the final volume within each well was adjusted to 200 µL. Plates were incubated for 15 h at 37°C and luciferase activity was determined in a multiplate reader (FLUOstar OPTIMA, BMG LABTECH). For that purpose 10 µL of D-luciferin stock solution (20 mM) were added to each well and measurement was started after 5 min of preincubation. The signal gain was set to 3 800 and a 5 s shaking period was introduced before each cycle was started to avoid a limitation of oxygen for the luciferase reaction. Luminescence was detected in three cycles using a photon detection time of 5 s per well and the sum of the three cycles was calculated from the raw data of the output analysis report. A blank containing medium with and without the addition of cycloheximide, which was not inoculated with conidia, was taken as a control and substracted from the raw data. All experiments were performed in triplicate and standard deviations were calculated. As a control one of the luciferase producing *E. coli* strains was grown in autoinduction medium in the presence and absence of 50 µg/mL cyclophosphamide. OD₅₅₀ of the independent cultures was adjusted and luciferase activity was determined as described for the *A. fumigatus* cultures.

### I.9 Imaging of bioluminescence from animals, organs and fungal cultures

Images were acquired using an IVIS 100 system (Xenogen Cooperation, Alameda, USA) according to the manufacturer's instructions. Analysis and acquisition was performed using Living Image software version 2.6 and 3 (Xenogen/Caliper). During image acquisition, mice were anesthetised using a constant flow of 2.5% isofluorane mixed with oxygen using an XGI-8 Gas Anesthesia System (Xenogen), controlling the duration of anaesthesia. Images were acquired for 5 min with a binning of four for luminescent signals from the exterior of mice, whereas luminescence of internal organs during dissection or from cultures in plates was integrated for 1min. All other photographic parameters were held constant. Quantification of photons per second emitted by each organ was performed by defining regions of interest corresponding to the respective organ of interest, using the Xenogen software living image version 2.6 and 3 (Xenogen/Caliper). The presence of *A. fumigatus* within different organs was verified from dead animals by the bioluminescence from intact or homogenised organs and by histopathology of thin sections.

### I.10 Histopathology

Organs of interest (lungs, kidneys, spleen small and big intestines, brain, and stomach) were removed from mice and immediately fixed in 3.7% neutral-buffered formaldehyde, embedded in paraffin, and cut into 5-µm thick sections. For preparation of sections from the head in order to analyse the sinus, skulls of mice were decalcified in a solution of 4% buffered formalin and 10% trichloroacetic acid for approximately 2 month. Serial 5-µm sections were stained with hematoxylin and eosin and with methenamine silver for fungal detection and examined microscopically(Sinha et al. 1988)

### II - Results

### II.1 Determination of the gpdA open reading frame and recombinant synthesis of glyceraldehyde-3-phosphate dehydrogenase

To proof that the putative *gpdA* gene indeed codes for the glyceraldehyde-3-phosphate dehydrogenase, RNA from peptone grown wild-type *A. fumigatus* mycelium was isolated and first strand cDNA was amplified by using anchored oligo(dT)₂₀ primers. The *gpdA* coding region was amplified from this cDNA which resulted in a single band of 1,021 bp. After subcloning of the fragment four independent clones were selected for sequencing. Analysis of the sequencing reaction revealed that the open reading frame of the *gpdA* gene deposited in the CADRE database was not correctly assigned. The intron No. 2 of the gene (Afu5g01970) does not consist of a single intron rather than of two introns with each 61 bp in length, separated by a short exon sequence of 14 bp. In addition the second of these two introns ended 14 nucleotides further downstream than annotated for intron 2 of Afu5g01970, which, therefore, leads to exactly the same transcript size but an altered amino acid sequence in the N-terminal region. Furthermore, the Inventors found a base exchange at position 816 of the coding sequence, which is a conservative exchange in a wobble position (AAA instead of AAG; both coding for lysine), which may be strain dependent. The correct coding sequence of the *gpdA* gene can be found under the accession number XYZ. The *gpdA* cDNA was subcloned into a pET expression vector. BL21 (DE3) Rosetta2 cells were used for enzyme production and a strain with an empty vector served as a control. After growth of the cells in overnight TB instant express medium crude extracts were prepared and tested for GpdA activity. The control cells displayed a significant activity of 0.92 U/mg, which can be mainly attributed to glyceraldehyde-3-phosphate dehydrogenase from *E. coli.* This activity was increased to 22.3 U/mg in the clones which contained the *gpdA* expression construct. After purification by Ni-chelate chromatography the purified protein displayed a specific activity of 27.55 U/mg. When arsenate was omitted from the assay only 80% of the maximum activity was reached, which is in good agreement with observations on other glyceraldehyde-3-phosphate dehydrogenases (Brenneman and Volk 1959). Electrophoresis of the samples in a 4 - 12% SDS-polyacrylamide gel revealed a single band with an apparent molecular mass of approximately 35 kDa (figure 1). Tryptic digestion and peptide analysis identified the GpdA coding gene from the NCBI database database but the overall score was not significantly high, which was mainly due to the fact that the N-terminal sequence of Afu5g01970 was not correctly assigned. Sequencing of a C-terminal peptide, however, revealed a score of 84.4, which unambigously identified the protein. When the sequence of the cDNA was used as template 41.1 % sequence coverage was obtained. From these results the Inventors conclude that the predicted *gpdA* gene indeed codes for the glyceraldehyde-3-phosphate dehydrogenase, although the annotation of the ORF of Afu5g01970 from the CADRE database needs correction.

### II.2 Construction of firefly luciferase producing A. fumigatus strains

In order to monitor the manifestation of invasive aspergillosis caused by *A. fumigatus* in live animals, the Inventors aimed in the construction of a bioluminescent pathogenic strain. Since bacterial luciferase operons have not been adapted for the use in eucaryotic systems, they utilised the *luc*-gene from the firefly *Photinus pyralis* with the aim to construct a strain, which displays a constitutive expression of the *luc*-gene. Since only limited data are available on the nutrient consumption of pathogenic fungi during tissue invasion, the promoter region of the putative glyceraldehyde-3-phosphate dehydrogenase (GpdA) was selected. GpdA is an enzyme which acts in both, the glycolysis as well as in gluconeogenesis. It catalyses the interconversion of glyceraldehyde-3-phosphate and 1,3-bisphosphoglycerate. At least either one of both pathways was assumed to be indispensible for the pathogen, because glucose has to be produced in its absence from the growth medium for shunting the pentose phosphate pathway, which is required for the reduction of NADP for oxidative processes and for providing ribose sugars, which are essential for the synthesis of nucleic acids and several cofactors. Investigations on the pathogenic yeast *C. albicans* revealed that enzymes involved in both, glycolysis as well as gluconeogenesis are, although differentially produced in different stages of pathogenesis, essential for full virulence, confirming the importance of both pathways during pathogenesis (Barelle et al. 2006).

The luciferase gene, optimised for eucaryotic expression, was obtained from the plasmid pSP-luc⁺ (Promega) and used for a fusion in frame with the ATG start codon downstream of the *gpdA* promoter. As a resistance marker for transformation of *A. fumigatus* the pyrithiamine resistance cassette was used. The cassette was amplified by PCR to generate *Not*I restriction sites at both ends and subcloned into the *PgpdA::luc* containing vector. The resulting plasmid PgpdA::luc/pJET/ptrA was used for transformation of the *A. fumigatus* wild-type strain CBS144.89. In an initial screening 32 transformants were streaked repeatedly on pyrithiamine containing media to ensure the selection of strains without contamination of wild-type nuclei. From these 32 strains six were randomly selected and tested for luciferase production. For that purpose mycelia were grown for 20 h in glucose minimal media, harvested, disrupted by grinding in liquid nitrogen and resuspended in buffer. From each sample 100 µg and 300 µg of total protein were applied to a luciferase assay. As a control different amounts of purified luciferase were used. In order to visualise a quenching effect on light production by the fungal protein extract a lysate of the wild-type strain CBS 144.89 was prepared and added to the standard control reactions. The luminescence was measured by a microplate reader (FLUOstar OPTIMA; BMG Labtech) and, in addition, a photo was taken with a digital camera (Minolta Dimage 7i) as shown in figure 2A. Intensity of light emission was in the following order: C3 > C8 > B4 ≥ B8 > B2 > C5. To proof the *in vivo* activity of light emission the parental wild-type strain and the strains C8, C3 and B4 were inoculated in 24 well plates and incubated for 8 h at 37°C in RPMI medium to allow the formation of germ tubes. D-Luciferin was added to each well and luminescence was counted by an IVIS light detection system (figure 2B) and quantified with the living image software (2C). In agreement with the determination of luminescence from crude extracts the light emission was stronger with strains C3 and C8 compared to B4. Furthermore, light emission correlated to the amount of conidia used to inoculate the media. Therefore, read outs by the IVIS system give, at least, a semi quantitative measure for the mycelial burden, which is important for quantification of the fungal burden in infected tissues.

### II.3 Suitability of the luciferase producing A. fumigatus strains for drug efficiency testing

To test the suitability of the luciferase producing *A. fumigatus* strains C3 and C8 for monitoring of the *in vitro* effectiveness of antifungal compounds, the Inventors used cycloheximide as a model compound, because it inhibits growth by disabling peptide chain elongation in protein synthesis of eucaryotic but not procaryotic cells (Baliga et al. 1969). As a control one of the luciferase producing *E. coli* strains was inoculated in the presence and absence of cycloheximide. Bacterial growth was hardly inhibited and detection of light emission by a microplate luminescence reader confirmed that cycloheximide had no negative effect on light emission (data not shown). This indicates that the drug neither inhibits the luciferase itself nor the light emitting reaction. Therefore, the system appeared suitable to correlate the fungal growth inhibition by cycloheximide with the respective luciferase activity.

**Table 3: Effect of antibiotics on growth and light emission from the luciferase producing A. fumigatus strain C3. A: Cycloheximide B: Nystatin**

| **A** | | | | |
|---|---|---|---|---|
| **Strain** | **Concentration** **[µg/mL]** | **Microscopic growth analysis^{a}** | **Relative response units^{b}** | **Relative activity** |
| C3 | 0 | 4.94 | 1.05 × 10⁶ ± 5.22 × 10⁴ | 100% ± 5.0 |
| C3 | 2.5 | 4.00 | 5.84 × 10⁵ ± 5.80 × 10⁴ | 56% ± 5.1 |
| C3 | 5.0 | 3.02 | 4.02 × 10⁵ ± 2.01 × 10⁴ | 38 % ± 2.2 |
| C3 | 10 | 2.57 | 1.88 × 10⁵ ± 1.02 × 10⁴ | 18% ± 0.9 |
| C3 | 25 | 1.48 | 8.31 × 10³ ± 4.54 × 10² | 0.8% ± 0.03 |
| C3 | 50 | 1.04 | 8.21 × 10² + 1.97 × 10² | 0.08% ± 0.02 |

| **B** | | | ^{c} | |
|---|---|---|---|---|
| C3 | 0 | 4.84 | 3.02 × 10⁵ ± 7.94 × 10³ | 100 ± 2.6 |
| C3 | 0.5 | 4.78 | 2.54 × 10⁵ ± 1.34 × 10⁴ | 84 ± 4.8 |
| C3 | 1.0 | 4.34 | 1.91 × 10⁵ ± 2.81 × 10³ | 63 ± 1.2 |
| C3 | 1.5 | 2.32 | 9.54 × 10⁴ ± 4.32 × 10³ | 32 ± 1.0 |
| C3 | 2.0 | 1.90 | 6.35 × 10⁴ ± 5.89 × 10² | 21 ± 0.2 |
| C3 | 3.0 | 1.08 | 4.10 × 10⁴ ± 3.17 × 10³ | 14 ± 0.6 |

| | | | | |
|---|---|---|---|---|
| ^{a} Microscopic growth analysis after incubation in the presence of antibiotics. Classification was performed from at least 100 conidia from each condition and numbers denote the average value of fungal outgrowth (5 = branching hyphae; 4 = elongated hyphae without branching; 3 = germ tubes; 2 = swollen conidia; 1 = resting conidia). All experiments were performed in triplicate and standard deviations for relative light emission are given. ^{b} Relative response units are given as the sum of 3 cycles á 5 s taken after 5 min of pre-incubation. ^{c} Relative response units are given as the average of 2 s intervals taken between 4 and 10 s directly after D-luciferin addition. | | | | |

As shown in table 3, a strong light emission was observed when no cycloheximide was present and nearly all conidia were germinated and formed branched hyphae. The addition of low amounts of cycloheximide (2.5 µg/ml) still allowed germination of conidia but hyphal length was decreased and branching was less frequently observed. The further increase of the cycloheximide concentration revealed a dose dependent inhibition of the germination rate and at 50 µg/ml hardly any conidium was swollen or germinated. In agreement with that, the light emission from these wells strongly decreased and at 50 µg/ml the luciferase activity was reduced by a factor of 1,000.

In a second approach we tested the antifungal drug Nystatin, which is frequently used to combat fungal superficial infections, because it is not absorbed by the skin and the intestine. In order to easily determine the maximum light emission in the absence of inhibitors, we changed the detection method by automatically injecting the D-luciferin to the wells directly prior to each measurement and started to record the data in short intervals of 2 s. Comparison of the data from 16 intervals revealed that the highest and most stable luminescence signals were obtained in a time frame between 4 and 10 s after injection of D-luciferin and the average of these four intervals was taken for comparison. Table 1B shows the correlation between fungal growth and light emission. Only a minor effect on growth and light emission was observed in the presence of 0.5 or 1.0 µg/ml of Nystatin. However, the further increase of the Nystatin concentration strongly delayed fungal germination and at 3 µg/ml nearly all conidia stayed in the resting state, which was resembled by a strong decrease in the luminescence signal.

From these experiments we conclude that luciferase production correlates with the growth inhibitory effect mediated by both drugs. Furthermore, this method also allows MIC determinations, which can be performed by graphical analysis of the luminescence signals (figure 3A and B). By defining 1% of residual light emission in comparison to the control as a suitable MIC value, it would require 29.6 µg /ml of cycloheximide and 6.5µg/ml of Nystatin to achieve this goal.

From this experiment the Inventors conclude that luciferase production correlates with the growth inhibitory effect of cycloheximide and that, therefore, the luciferase producing strains may be suitable, in a general approach, to prescreen the *in vitro* efficiency of antifungal compounds.

### II.4 Establishment of invasive pulmonary aspergillosis in corticosteroid treated BALB/cJ mice

For all animal experiments the bioluminescent strain C3 was used, which showed the strongest luminescence signal in the *in vitro* experiments. Mice were immunosuppressed by two single doses of cortisone acetate three days prior and at the day of infection. As a control D-luciferin was injected intraperitoneally to uninfected immunosuppressed mice and luminescence was measured by the IVIS system. Despite some weak background over the whole mice, which was only visible in high sensititvity measurements, no specific organ highlighted in this experiment. In the first *in vivo* BLI experiment 2 × 10⁶ conidia were applied to the nares of two anaesthetised BALB/cJ mice in order to confirm the experimental setup for subsequent experiments and to gain first insight onto the development of invasive pulmonary aspergillosis. Both mice started to develop signs of illness such as ongoing weight loss, lethargy and, in later stages, a pulmonar distress. Coinciding with that luminescence appeared from the region of the lung and was detectable until death of the animals at days three and four. When organs from these mice were removed, homogenised and analysed by bioluminescence imaging, only the lungs showed strong luminescence (data not shown), whereas other organs such as liver, kidney and brain remained negative for light emission. To get a better overview on the establishment and manifestation of infection by distinct conidia concentrations groups of three to five mice were infected with different conidia concentrations in the following experiments.

### II.5 Intranasal infection of corticosteroid treated BALB/cJ mice with 2 × 10⁶ and 7 × 10⁷ conidia

In order to confirm the suitability of the luciferase producing *A. fumigatus* strain C3 to monitor the progression and localisation of infection in dependency on the conidia concentration applied, the Inventors infected four BALB/cJ mice with 2 × 10⁶ conidia and five mice with 7 × 10⁷ conidia. All mice were treated with cortisone acetate prior to infection.

Upon infection with 2 × 10⁶ conidia, all mice displayed luminescence from their lungs 23 h after infection, when D-luciferin was injected intraperitoneally. Luminescence stayed visible until death of animals. The lungs from mice No. 1, 4 and 5, which were removed post mortem, displayed strong luminescence after direct injection of D-luciferin. All mice died within four days after infection. Upon infection with 7 × 10⁷ conidia, strong luminescence appeared 18 h after infection and was detectable until death of animals. No luminescence was observed, when dead animals were treated intraperitoneally with D-luciferin. Direct injection of D-luciferin into the single organs revealed only a bright luminescence signal for the lungs, whereas all other organs remained negative.

In all cases mice developed an invasive pulmonary aspergillosis and died within three to four days after infection. The most striking difference in both experiments was that the luminescence signal peaked at around 29 h when mice were infected with the lower dose of conidia, whereas the peak signal was already observed after 18 h when mice were infected with 7 × 10⁷ conidia . Furthermore, luminescence was much brighter when the higher dose of conidia was applied, coinciding with a dependency of light emission on the amount of germinating hyphae present (data not shown). No light emission was observed from dead animals, which was due to the missing transport of intraperitoneally injected D-luciferin to the site of infection. When such a mouse was opened without further injection of D-luciferin some light emission was observed, which was enhanced by a factor of 21 when D-luciferin was directly injected into the lung. However, no other organs showed any light emission when D-luciferin was injected, indicating that invasive aspergillosis was restricted to the lung. This observation was confirmed by histopathology, in which all other organs remained negative for fungal burden, whereas all lungs were heavily infected (data not shown). Histopathology furthermore revealed that the overall fungal burden in the lungs of mice infected with the higher dose of conidia was strongly increased in comparison to mice infected with the lower dose. HES staining of the tissue of mice infected with the higher dose also showed that all lesions were highly infiltrated by granulocytes and large necrotic areas where observed throughout the lung, which explains the pulmonary distress observed. An independent repetition of the intranasal infection yielded comparable results (not shown), which implicates that the model system is suitable to monitor disease development. All mice investigated here were negative for fungal dissemination, indicating that mice die before an infection can manifest within secondary organs.

### II.6 Intranasal infection of corticosteroid treated Swiss OF1 mice with 7 × 10⁷ conidia FIG 6

In various laboratories quite often different mouse strains are used to study the virulence of *A. fumigatus* mutant strains. To test whether mouse strains behave differently in respect to disease development, the Inventors additionally used Swiss OF1 mice to monitor the establishment and progression of invasive aspergillosis. For this purpose, they applied 7 × 10⁷ conidia to five cortisone acetate treated mice.

This treatment led to death of all animals within 6 days after infection. Although luminescence within lung tissue was observed in all cases, the peak values and distribution of luminescence differed between individual mice.

The differences in luminescence development between single mice early after infection indicate that not all mice received the whole load of conidia within the pulmonary tract. Mice No. 2 and 4, which died after 48 and 69 h, respectively, displayed strongest luminescence from the lung and from the trachea, as can be seen after removal of the lung from mouse No. 4. Mouse No. 5 not only developed luminescence from the lung (14 h post infection) but also displayed light emission from the stomach (25 h after infection until death). Mice No. 1 and 3 showed increasing luminescence from the sinus region, which was best visible in the dorsal views at 64, 72 and 88h and only show weak signals from the lung. Injection of luciferin to all organs after death of the animals confirmed that no other organs except lung, sinus, trachea or stomach showed light emission.

A more detailed look revealed that three subtypes of infectious disease became visible, which will be outlined below. **Subtype I:** Mice No. 2 and 4 fall into this class. Both mice displayed an extremely high peak of luminescence from the lungs already 14 hours after infection. In this case, the major part of conidia had reached the pulmonary tract and the comparison to the BALB/cJ mice infected with the same amount of conidia showed no significant differences. Due to the rapid outgrowth of the conidia an early respiratory distress was observed and mice died within 64 h post infection. Although an early peak in luminescence was also observed at the sinus region, the infection did not manifest within this locus since luminescence rapidly declined. Histopathologic sections clearly showed that lungs were heavily infected and a huge number of foci with necrotic tissue and a high fungal burden was visible. Interestingly, some luminescence was also observed from the digestive tract of mouse No. 2, especially at the region of the stomach and appeared two days after infection. However, since this mouse died view hours later and luminescence from this region was much less pronounced than in mouse No. 5, this finding will be discussed below. **Subtype II:** This type was mainly represented by mice No. 1 and 3. Both mice displayed luminescence from the lung but light emission peaked later and much less dramatically when compared to subtype I. Nevertheless, lung luminescence was present until death of mice at days four and six, respectively. Histopathologic sections showed that infected lesions were present in the lung but their number was strongly reduced in comparison to subtype I. Furthermore, single lesions seemed to derive from less conidia than observed in the former subtype. In addition to the luminescence from the pulmonary tract, both mice revealed strong luminescence at the sinus region, which did not decline after the first two days rather than becoming more intensive over time. Moreover, coinciding with increasing sinus luminescence, both mice developed a disturbance of equilibrium, which was indicative for a neural dysfunction mediated by the fungal infection. Histopathologic head sections from both mice confirmed this assumption. Invasive fungal growth was observed all over the sinus region and, although the brain itself did not show any fungal burden , the equilibrium organ might have become affected by the fungus. **Subtype III:** This subtype is mainly represented by mouse No. 5 and to a lesser extend in mouse No. 2 and was also observed in an independent repetition of the experiment. This type was and intermediate between subtypes II and I. In subtype III the pulmonary tract, the sinus region and the stomach were severely affected. Luminescence of the lung peaked at day two, which indicates that a significant proportion of conidia were deeply inhaled. This was furthermore confirmed by the histopathology of lung sections, in which a slightly lower fungal burden was observed compared to histopathology of mice No. 2 and 4 . Mouse No. 5 also showed significant luminescence from the sinus region indicating a severe sinusitis and the mouse died when disturbance of equilibrium occurred (88 h post infection). The luminescence from the stomach, which was highly abundant 41 hours after infection, stayed at a high level until death of the mouse. A histopathologic investigation of this organ revealed outgrowing hyphae, which, although looking somewhat stunted, invaded the mucus of the stomach . This leads to the assumption that the treatment with steroids also alters the ability of the intestinal tract, at least of some individual animals, to digest swallowed conidia. This observation was very interesting in light of the acidic environment present within the stomach, which was supposed to prohibit outgrowth of *A. fumigatus* conidia. However, the genome of *A. fumigatus* contains at least one gene coding for a urease (AFUA_1G04560), which might contribute to resistance against acid stress but has not been investigated in more detail.

Although in this experiment conidia were able to germinate at different sites than observed with the BALB/cJ, there was no evidence for a systemic dissemination via the blood stream. Furthermore, although in some cases a lower amount of conidia reached the lungs, all Swiss OF1 mice displayed a constant weight loss of approximately 2 grams per day, which confirmed a severe infection caused by *A. fumigatus* and mice finally died or had to be sacrificed because of the physical distress.

### II.7 Intravenous infection of BALB/cJ and Swiss OF1 mice (data not shown)

Since the luminescent *A. fumigatus* strain turned out to be suitable to monitor disease development after intranasal application of conidia, the Inventors were interested, whether disseminated aspergillosis could also be monitored by BLI. For this purpose mice were immunosuppressed with cortisone acetate as described above and infected *via* the lateral tail vein with 2 × 10⁶ conidia. In case of BALB/cJ five and in case of Swiss OF1 three mice were used. In all cases luminescence accumulated in the early phase of infection (2.5 - 7 h) within the chest region, indicating that spores were transported to the heart and lung. After 18 to 23 h the luminescence increased dramatically within the liver as seen in the ventral view from both mouse strains. In addition, at this time point both kidneys of each animal became strongly visible in BLI in the dorsal view (data not shown). This indicates that conidia were filtered off in these organs and germinated within both tissues. In both experiments most of the mice died within 48 h with the exception of mouse No 4 of the BALB/cJ strain, which survived for 69 h. When mice were opened, especially the lung and heart, the liver and both kidneys were extremely luminescent (date not shown). Other organs such as the brain or the spleen only showed week luminescence, indicating that these organs were not the preferred sites for fungal outgrowth although luminescence was slightly elevated in comparison to the control. When the luminescence signals of the removed organs from the Swiss OF1 mouse No.1 were compared with the luminescence of organs from a PBS treated control mouse the following increase in luminescence was determined: Brain = 8 times, lung + heart = 327 times, spleen = 30 times, liver = 250 times, left kidney = 1588 times and right kidney = 1376 times. Histopathology of the organs again revealed a very good correlation between the luminescence signal and the fungal burden within these tissues.

A hematoxylin eosin staining confirmed the outgrowth from the blood vessels. An agglutination of erythrocytes occurred within the blood vessels especially in the liver and the kidney.

In contrast to the inhalation model, in which hyphae started to grow within the alveolar spaces of the lung, the tissues were always infected *via* crossing of hyphae through the wall of blood vessels, when conidia were applied intravenously.

### II.8 Pathogenesis of aspergillosis following different immunosuppression regimen

Fig 10 shows the survival curve of immunosuppressed BALB/c mice infected intranasally either with 2 × 10⁵ or 2 × 10⁶ conidia. At the highest concentration and under the different immunosuppression regimen, all the animals die between 4 and 4.5 days post infection. 20% of animals infected with 2 × 10⁵ survived within this range and 100% mortality is obtained after 7 days of infection. The susceptibility to infection of each group was also followed up by the loss in weight (Fig11). Cyclophosphamide treated mice show the highest 32 % of loss with a moribund shape compared to 30 and 28% of loss observed for animals immunosuppressed with cortisone acetate after infection with 2 × 10⁵ or 2 × 10⁶ conidia. RB6 treated animal show only 14% weight-losses.

### Fig13a: Comparison after D1 infection

As shown in Fig 13b, examination of hemalun-stained lung sections from the different groups of animals died between D3 and D4.5 days shows different patterns of the inflammatory response. Cortisone acetate (2.10⁶ conidia): (a-b) Multifocal inflammatory lesion, centered on bronchi/bronchioles but secondly extending to alveoli and blood vessels (veins and arteries), displaying a concentric organisation. In contrast to the 2.10⁵ dose of infection, fungi are not limited to bronchial/bronchiolar spaces; they could indeed infiltrate bronchiolar walls and spread to peripheral alveoli (arrows). (c) In the centre, bronchiolar, alveolar and vascular (arrowhead) spaces are infiltrated mostly by karyorrhectic neutrophils: suppurative bronchopneumonia and leucocytoclastic vasculitis. They are circled by a peripheral rim of macrophages, sometimes displaying an abundant, acidophilic cytoplasm and a vesiculous, euchromatic and nucleolated nucleus (epithelioid cells; arrowhead): pyogranulomatous lesion (d). RB6 (2 × 10⁶ conidia): (a) like the cortisone acetate treatment, a multifocal inflammatory lesion, centred on bronchi/bronchioles but secondary extending to alveoli and blood vessels is observed. It is however a more severe lesion characterized by larger areas of necrosis, more severe vascular lesions (vasculitis and haemorrhages) and a stronger infiltration of alveoli by inflammatory cells and fungi (b). In contrast to the cortisone acetate treatment, the inflammatory infiltrate is almost strictly composed of mononucleated cells (macrophages and few lymphocytes and plasma cells). Rare eosinophils can nevertheless be observed (arrowheads) (c).

Cyclophosphamide (2 × 10⁶ conidia): (a) Multifocal to coalescing lesion
characterized by a total lack of inflammatory response and (b-d) a fungal severe and extensive infiltration of the pulmonary parenchyma and vascular walls and spaces (stars), leading to thrombosis and infarcts. (c) Bronchial/bronchiolar and alveolar epithelial cells display a loss of their nuclei and a clumping of their cytoplasm, but with preservation of basic outlines of alveolar architecture (ischemic necrosis).

### Monitoring of the fungal burden with a luminescent C3 strain of A. fumigatus

The luciferase gene, optimised for eukaryotic expression, was placed under control of A. fumigatus *gpdA* promoter. The resulting plasmid PgpdA::luc/pJET/ptrA (see supplement) was used to transform the *A. fumigatus* wild-type strain CBS144.89. Six randomly chosen transformants (out of 32) were selected as previously described. Among them the transformant C3 has been chosen for further analysis and tested for luciferase production (Ref). The light emission correlated with the number of conidia used to inoculate the media (data not shown). Therefore, read outs by the IVIS system give, at least, a semi quantitative measure for the mycelial burden, which is important for quantification of the fungal burden in infected tissues.

As shown in Figure 12, each point represents the average of total photon flux measured from a defined chest region of interest from each individual animal. After 24h infection the highest luminescence was visible in the chest area of the cortisone acetate treated mice. The peak of luminescence was observed after 2D of infection in the RB-6 treated animals. The cyclophosphamide treated animals show a delayed luminescence starting to increase from D2 with the highest value at D4. These observations fit with the inflammatory response as observed in the mice sections (Fig 13a). At the time of death, sections either from the cortisone acetate or RB6-treated displayed high necrosis and suppurative lesions with fungi present in the bronchial/bronchiolar spaces and extending to alveoli and blood vessels. As a consequence of this necrosis and haemorrhage, oxygen can barely be delivered to the site of infection making difficult the enzymatic activity of the luciferase. This limitation can be circumvented in the cyclophosphamide treated animals where any necrosis could be observed.

To better evaluate the early inflammatory response additional experiment has been done under the same conditions but animals have been sacrificed for histology after 1D infection. As for the previous experiment, the luminescence values were respectively, of 3,86E+05, 1,48E+05 and 1,12E+05 for cortisone acetate, cyclophosphamide and RB-6 treated animals .

Taking altogether these experiments enabled us to suggest a model for the early and late inflammatory response against *A. fumigatus* based on the bioluminescence data reflecting the degree of growing hyphae withing the lungs and the mice sections.

### III - Discussion

In this study, the Inventors constructed a bioluminescent *A. fumigatus* strain, which turned out as an excellent tool to follow the temporal and spatial development of invasive aspergillosis since the strain displayed no reduced virulence in comparison to the parental strain CBS144.89. For the construction of the bioluminescent strain the sequence coding for the glyceraldehyde-3-phosphate dehydrogenase was determined and functionality of the corresponding protein was confirmed by heterologous production in *E. coli.* By that means the Inventors recognised that the computational mRNA prediction of the *gpdA* gene contained a wrong intron annotation within the 5'-coding region. However, the start codon showed the correct prediction and they used the upstream flanking region as a promoter for constitutive luciferase production.

*In vitro* experiments confirmed that the resulting *A. fumigatus* transformants emitted light under all growth conditions applied (only examples shown). Since they observed a correlation between outgrowth of conidia and light emission, they used two of the transformants for testing the efficiency of the model antibiotic cycloheximide in concentration dependent growth inhibition. The results showed that as little as 2.5 µg/mL inhibited growth and light emission of both strains and 50 µg/mL hardly allowed swelling of conidia and light emission was reduced by a factor of 1000. Therefore, they conclude that bioluminescence provides a suitable tool for rapid screening of new antifungals for their growth inhibitory effect. Nevertheless, the method could be improved by automatic injection of D-luciferin directly prior to luminescence detection from the respective well. This should minimise the standard deviation from the single measurements, which was enforced by the reduction in light emission due to the consumption of D-luciferin prior to the data collection at wells with high luciferase activity.

Besides the suitability of the luciferase producing strains in *in vitro* experiments, the strains were well suited for studying disease development in living mice. Luminescence and histopathology coincided from all body sites, which confirmed the Inventors' former hypothesis that either gluconeogenesis or glycolysis should be active during invasive fungal growth. Since an importance of a functional glycolysis and gluconeogenesis for full virulence was already asserted for the pathogenic yeast *C. albicans* (Barelle et al. 2006), a similar dependency for these pathways may also come true for *A. fumigatus.*

In contrast to earlier investigations on *C. albicans,* in which the firefly luciferase under the control of the *ENO1* promoter was used to study fungal dissemination in murine infection models, the Inventors were not faced with the problem that light emission was too low to detect the fungus within the living mouse (Doyle et al. 2006a). In addition, taking the evaluation of the histopathologic sections as an independent measure for the fungal burden in different organs, they found a good correlation between light emission and amount of mycelium, which was not related, when light emission in disseminated candidiasis was compared with colony forming units (Doyle et al. 2006a). Therefore, the Inventors' system provides the first suitable model for *in vivo* monitoring of an invasive fungal infection by use of a pathogenic luciferase producing *A. fumigatus* strain.

However, one drawback of the luciferase system, which is not a problem specifically, emerging from their investigation, was the strict dependency on a sufficient oxygen supply for the light emitting reaction. The lung tissue was generally assumed as an organ in which oxygen is not limited because of the permanent exchange of air with the environment. This assumption seemed to be true at least during the early onset of an invasive pulmonary aspergillosis. Intranasal infection with conidia led to a strong light emission already one day after infection, regardless the mouse strain used. However, the Inventors noticed that the luminescence signal peaked after one to two days, turned into a plateau phase for approximately one day and declined later on although the fungal burden was assumed to increase, which was indicated by the ongoing weight loss of mice and the steadily decreasing fitness of the animals. The reason for this phenomenon is the extreme weak clinical state of the animals avoiding the delivery o luciferin . Mice infected with the high dose of 7 × 10⁷ conidia, which showed a very high luminescence signal early in infection, revealed necrotic areas throughout the lung. Since necrotic areas can also form anaerobic niches for strictly anaerobic pathogenic bacteria causing pleuromopulmonary infections (Bartlett 1993; Levison 2001), the oxygen supply at the site of fungal growth might become extremely restricted. However, studies focusing on the anaerobic or microaerophilic growth behaviour of *Aspergillus* species are very limited with the exception of one report, in which the strict aerobic fungus *Aspergillus nidulans,* a close relative of *A. fumigatus,* was grown anaerobically in the presence of nitrate and ethanol, whereby nitrate was reduced to ammonia and ethanol oxidised to acetate (Takasaki et al. 2004). It remains questionable, whether such pathways also play a role in A. *fumigatus* pathogenesis but, at least, survival under anaerobic and microaerophilic conditions should be studied in the future.

When intranasal application of conidia and disease development were compared between the two mouse strains Swiss OF1 and BALB/cJ, the Inventors noticed an interesting difference. Although they were aware that various mouse strains might display different prevalences for certain infectious diseases, they did not expect to observe the manifestation of invasive fungal growth at different body sites. However, in BALB/cJ mice intranasal infection always led to fungal growth restricted to the bronchopulmonary tract and infection of the sinus region did not seem to occur frequently. In this study they finaly infected more than 15 BALB/cJ mice intranasally and never observed a manifestation of infection at the sinus. In contrast, sinusitis caused by invasively growing hyphae was observed with the Swiss OF1 strain and mice displaying a persisting luminescence from the head for more than three days started to develop a disturbance in equilibrium, continued in weight loss and either died from the infection or had to be sacrificed because of the bad physical status. This was never observed with the BALB/cJ mice, indicating that, at least in some individuals, the Swiss OF1 background caused an increased susceptibility for disease development within the sinus. In addition, in three out of 15 Swiss OF1 mice infected intranasaly they observed luminescence deriving from the stomach and histopathology did not reveal any gastritis lesions in the stomach tissue. Although the disturbance in equilibrium has not been frequently reported before, it would be interesting to investigate, whether some mouse strains display an increased susceptibility for sinus infections. Furthermore, the Inventors were able to visualise the drawback of the intranasal infection by using conidia suspensions, since the strength of anaesthesia seems to be crucial for infecting the lungs with high conidial loads. This problem may be circumvented by using an inhalation chamber in which conidia are nebulised and inhaled by none-anaesthetised mice (Sheppard et al. 2006). However, after treatment within the inhalation chamber only lungs were investigated for theirconidial loads and huge amounts of conidia may either stay within the sinus region or may become swallowed after the general infection process when mice clean up their coat. Therefore, infections of the sinus may contribute to death of mice.

Intravenous infection is not assumed to resemble a general route for the acquisition of invasive aspergillosis. Nevertheless, this way of infection was frequently used when the efficiency of antifungal drugs was tested (Odds et al. 1998; Takemoto et al. 2004; Sionov et al. 2006). Therefore, the Inventors were interested in the distribution of invasively growing hyphae in the different tissues. Since the intranasal infection in the mouse model only led to a restricted distribution to the lung, the sinus region and, in some cases, the stomach, they also wanted to proof, whether luminescence could be detected from other organs such as the liver and the kidneys. BLI revealed that after an initial phase, in which conidia were transported to the heart, the infection mainly manifested within the liver and the kidneys. This may be caused by the general function of these organs in filtering blood. However, the interesting observation from histopathology was that the vascular wall was disrupted by the fungus, which allowed the entry of hyphae into the tissue. In the inhalation model of mice, however, the animals most likely died from lung infarction due to blood vessel destruction prior to manifestation of infection in other organs. This hypothesis may explain, why dissemination to other organs was never observed in the inhalation infection model.

In conclusion, BLI was identified as an excellent tool to study the growth inhibitory action of an antifungal drug *in vitro* and the manifestation and progression of invasive aspergillosis *in vivo.* Thereby, this investigation provides the first example in which the luciferase system was succesfully used to monitor development of a fungal infection *in vivo.* The use of the *gpdA* promoter enabeled the determination of fungal burden from all infected body sites. No false positive detection was obtained and background luminescence was neglegtibly low. Therefore, the system may also be used to study the efficiency of antifungal drugs *in vivo,* as formerly performed in a vaginal infection model with *C. albicans* (Doyle et al. 2006a), but, in contrast to the *C. albicans* model, the system presented here should be applicable to all routes of infection. In addition, the Inventors were able to predict the amount of conidia entering the lung after intranasal spore application since the peak value of luminescence directly correlated with the fungal burden as determined from histopathologic sections. Conidia, which either stayed within the sinus, were able to cause sinusites and different mouse strains seem to possess different prerequisites for these infections. However, dissemination via the blood stream was never observed. Although liver and kidneys are not the primary targets in disease development in humans, mycelium was sometimes detected at these sites when patients died from aspergillosis. Their intravenous infection model revealed that mycelium was always highly abundant in the liver and kidneys, whereas the amount within the heart and lung varied to some extend and spleen and brain only displayed small amounts of fungal burden. Therefore, the former organs seem to provide excellent conditions for invasive fungal growth and the observation of liver and kidney infections may generally resemble dissemination *via* the blood stream.

### BIBLIOGRAPHIC REFERENCES

Altiparmak MR, Apaydin S, Trablus S, Serdengecti K, Ataman R et al. (2002) Systemic fungal infections after renal transplantation. Scandinavian journal of infectious diseases 34(4): 284-288.
Baliga BS, Pronczuk AW, Munro HN (1969) Mechanism of cycloheximide inhibition of protein synthesis in a cell-free system prepared from rat liver. The Journal of biological chemistry 244(16): 4480-4489.
Ballance DJ, Turner G (1985) Development of a high-frequency transforming vector for Aspergillus nidulans. Gene 36(3): 321-331.
Balloy V, Huerre M, Latgé JP, Chignard M (2005) Differences in patterns of infection and inflammation for corticosteroid treatment and chemotherapy in experimental invasive pulmonary aspergillosis. Infection and immunity 73(1): 494-503.
Barelle CJ, Priest CL, Maccallum DM, Gow NA, Odds FC et al. (2006) Nichespecific regulation of central metabolic pathways in a fungal pathogen. Cellular microbiology 8(6): 961-971.
Bartlett JG (1993) Anaerobic bacterial infections of the lung and pleural space. Clin Infect Dis 16 Suppl 4: S248-255.
Brenneman FN, Volk WA (1959) Glyceraldehyde phosphate dehydrogenase activity with triphosphopyridine nucleotide and with diphosphopyridine nucleotide. The Journal of biological chemistry 234: 2443-2447.
Cardoza RE, Vizcaino JA, Hermosa MR, Monte E, Gutiérrez S (2006) A comparison of the phenotypic and genetic stability of recombinant Trichoderma spp. generated by protoplast- and Agrobacterium-mediated transformation. J Microbiol. Aug;44(4):383-95.
Crowhurst RN, Rees-George J, Rikkerink EH, Templeton MD (1992) High efficiency transformation of Fusarium solani f. sp. cucurbitae race 2 (mating population V). Curr Genet. May;21(6):463-9.
Dellacasa-Lindberg I, Hitziger N, Barragan A (2007) Localized recrudescence of Toxoplasma infections in the central nervous system of immunocompromised mice assessed by in vivo bioluminescence imaging. Microbes and infection / Institut Pasteur 9(11): 1291-1298.
Denning DW (1994) Treatment of invasive aspergillosis. The Journal of infection 28 Suppl 1: 25-33.
Doyle TC, Nawotka KA, Kawahara CB, Francis KP, Contag PR (2006a) Visualizing fungal infections in living mice using bioluminescent pathogenic Candida albicans strains transformed with the firefly luciferase gene. Microbial pathogenesis 40(2): 82-90.
Doyle TC, Nawotka KA, Purchio AF, Akin AR, Francis KP et al. (2006b) Expression of firefly luciferase in Candida albicans and its use in the selection of stable transformants. Microbial pathogenesis 40(2): 69-81.
Dubourdeau M, Athman R, Balloy V, Huerre M, Chignard M et al. (2006) Aspergillus fumigatus induces innate immune responses in alveolar macrophages through the MAPK pathway independently of TLR2 and TLR4. J Immunol 177(6): 3994-4001.
Francis KP, Joh D, Bellinger-Kawahara C, Hawkinson MJ, Purchio TF et al. (2000) Monitoring bioluminescent Staphylococcus aureus infections in living mice using a novel luxABCDE construct. Infection and immunity 68(6): 3594-3600.
Francis KP, Yu J, Bellinger-Kawahara C, Joh D, Hawkinson MJ et al. (2001) Visualizing pneumococcal infections in the lungs of live mice using bioluminescent Streptococcus pneumoniae transformed with a novel gram-positive lux transposon. Infection and immunity 69(5): 3350-3358.
Franke-Fayard B, Waters AP, Janse CJ (2006) Real-time in vivo imaging of transgenic bioluminescent blood stages of rodent malaria parasites in mice. Nature protocols 1(1): 476-485.
Glomski IJ, Piris-Gimenez A, Huerre M, Mock M, Goossens PL (2007) Primary involvement of pharynx and peyer's patch in inhalational and intestinal anthrax. PLoS pathogens 3(6): e76.
Gottfredsson M, Steingrímsdóttir H (2006) Disseminated invasive aspergillosis in a patient with acute leukaemia. Acta Biomed 77 Suppl 2: 10-13.
Hardy J, Francis KP, DeBoer M, Chu P, Gibbs K et al. (2004) Extracellular replication of Listeria monocytogenes in the murine gall bladder. Science (New York, NY 303(5659): 851-853.
Hasan RA, Abuhammour W (2006) Invasive aspergillosis in children with hematologic malignancies. Paediatric drugs 8(1): 15-24.
He ZM, Price MS, Obrian GR, Georgianna DR, Payne GA (2007) Improved protocols for functional analysis in the pathogenic fungus Aspergillus flavus. BMC Microbiol. Nov 26;7:104
Hutchens M, Luker GD (2007) Applications of bioluminescence imaging to the study of infectious diseases. Cellular microbiology 9(10): 2315-2322.
Ibrahim-Granet O, Dubourdeau M, Latgé JP, Ave P, Huerre M et al. (2008) Methylcitrate synthase from Aspergillus fumigatus is essential for manifestation of invasive aspergillosis. Cellular microbiology 10(1): 134-148.
Karsi A, Lawrence ML (2007) Broad host range fluorescence and bioluminescence expression vectors for Gram-negative bacteria. Plasmid 57(3): 286-295.
Latgé JP (1999) Aspergillus fumigatus and aspergillosis. Clinical microbiology reviews 12(2): 310-350.
Leroy P, Smismans A, Seute T (2006) Invasive pulmonary and central nervous system aspergillosis after near-drowning of a child: case report and review of the literature. Pediatrics 118(2): e509-513.
Levison ME (2001) Anaerobic pleuropulmonary infection. Current opinion in infectious diseases 14(2): 187-191.
Liebmann B, Mühleisen TW, Müller M, Hecht M, Weidner G et al. (2004) Deletion of the Aspergillus fumigatus lysine biosynthesis gene lysF encoding homoaconitase leads to attenuated virulence in a low-dose mouse infection model of invasive aspergillosis. Archives of microbiology 181(5): 378-383.
Montagnoli C, Fallarino F, Gaziano R, Bozza S, Bellocchio S et al. (2006) Immunity and tolerance to Aspergillus involve functionally distinct regulatory T cells and tryptophan catabolism. J Immunol 176(3): 1712-1723.
Odds FC, Van Gerven F, Espinel-Ingroff A, Bartlett MS, Ghannoum MA et al. (1998) Evaluation of possible correlations between antifungal susceptibilities of filamentous fungi in vitro and antifungal treatment outcomes in animal infection models. Antimicrobial agents and chemotherapy 42(2): 282-288.
Papadimitriou JM, Ashman RB (1986) The pathogenesis of acute systemic candidiasis in a susceptible inbred mouse strain. The Journal of pathology 150(4): 257-265.
Pasqualotto AC, Denning DW (2006) Post-operative aspergillosis. Clin Microbiol Infect 12(11): 1060-1076.
Saeij JP, Boyle JP, Grigg ME, Arrizabalaga G, Boothroyd JC (2005) Bioluminescence imaging of Toxoplasma gondii infection in living mice reveals dramatic differences between strains. Infection and immunity 73(2): 695-702.
Sarfati J, Diaquin M, Debeaupuis JP, Schmidt A, Lecaque D et al. (2002) A new experimental murine aspergillosis model to identify strains of Aspergillus fumigatus with reduced virulence. Nihon Ishinkin Gakkai zasshi = Japanese journal of medical mycology 43(4): 203-213.
Schaffner A, Schaffner T (1987) Glucocorticoid-induced impairment of macrophage antimicrobial activity: mechanisms and dependence on the state of activation. Reviews of infectious diseases 9 Suppl 5: S620-629.
Schöbel F, Ibrahim-Granet O, Ave P, Latgé JP, Brakhage AA et al. (2007) Aspergillus fumigatus does not require fatty acid metabolism via isocitrate lyase for development of invasive aspergillosis. Infection and immunity 75(3): 1237-1244.
Sheppard DC, Graybill JR, Najvar LK, Chiang LY, Doedt T et al. (2006) Standardization of an experimental murine model of invasive pulmonary aspergillosis. Antimicrobial agents and chemotherapy 50(10): 3501-3503.
Singh N, Paterson DL (2005) Aspergillus infections in transplant recipients. Clinical microbiology reviews 18(1): 44-69.
Sinha BK, Monga DP, Prasad S (1988) A combination of Gomori-Grocott methenamine silver nitrate and hematoxylene and eosin staining technique for the demonstration of Candida albicans in tissue. Quad Sclavo Diagn 24(1-4): 129-132.
Sionov E, Mendlovic S, Segal E (2006) Efficacy of amphotericin B or amphotericin B-intralipid in combination with caspofungin against experimental aspergillosis. The Journal of infection 53(2): 131-139.
Sjölinder H, Jonsson AB (2007) Imaging of disease dynamics during meningococcal sepsis. PLoS ONE 2(2): e241.
Stephens-Romero SD, Mednick AJ, Feldmesser M (2005) The pathogenesis of fatal outcome in murine pulmonary aspergillosis depends on the neutrophil depletion strategy. Infection and immunity 73(1): 114-125.
Takasaki K, Shoun H, Yamaguchi M, Takeo K, Nakamura A et al. (2004) Fungal ammonia fermentation, a novel metabolic mechanism that couples the dissimilatory and assimilatory pathways of both nitrate and ethanol. Role of acetyl-CoA synthetase in anaerobic ATP synthesis. The Journal of biological chemistry 279(13): 12414-12420.
Takemoto K, Yamamoto Y, Ueda Y, Sumita Y, Yoshida K et al. (2004) Comparative studies on the efficacy of AmBisome and Fungizone in a mouse model of disseminated aspergillosis. The Journal of antimicrobial chemotherapy 53(2): 311-317.
Woods JP, Heinecke EL, Goldman WE (1998) Electrotransformation and expression of bacterial genes encoding hygromycin phosphotransferase and beta-galactosidase in the pathogenic fungus Histoplasma capsulatum. Infect Immun. Apr;66(4):1697-707

## Claims

1. A nucleic acid comprising a gene encoding a luciferase which is under the control of a promoter for expression of said luciferase in a filamentous fungal host cell, advantageously an *Aspergillus fumigatus* host cell.

2. The nucleic acid according to claim 1, wherein the promoter is the glyceraldehyde-3-phosphate dehydrogenase promoter from *Aspergillus fumigatus,* which allows constitutive expression of the luciferase and has a sequence selected among anyone of the following sequences:
a) the sequence SEQ ID N°1,
b) a sequence having at least 70 % of identity with SEQ ID N°1 which retains the activity of the glyceraldehyde-3-phosphate dehydrogenase promoter from *Aspergillus fumigatus,* and
c) a sequence complementary to anyone of the sequences defined in a) and b).

3. The nucleic acid according to claim 1 or 2, wherein the gene encoding a luciferase is from the firefly *Photinus pyralis* and has a sequence selected among anyone of the following sequences:
a) the sequence SEQ ID N°2,
b) a sequence having at least 70 % of identity with SEQ ID N°2 encoding a protein which retains the activity of the firefly *Photinus pyralis* luciferase, and
c) a sequence complementary to anyone of the sequences defined in a) and b).

4. The nucleic acid according to anyone of claims 1 to 3, which comprises a sequence selected among anyone of the following sequences:
a) the sequence SEQ ID N°3 corresponding to the firefly *Photinus pyralis* luciferase which is under the control of the glyceraldehyde-3-phosphate dehydrogenase promoter from *Aspergillus fumigatus,*
b) a sequence having at least 70 % of identity with SEQ ID N°3 which retains the activity of the glyceraldehyde-3-phosphate dehydrogenase promoter from *Aspergillus fumigatus* and which encodes a protein which retains the activity of the firefly *Photinus pyralis* luciferase, and
c) a sequence complementary to anyone of the sequences defined in a) and b).

5. The nucleic acid according to anyone of claims 1 to 4, which further comprises the pyrithiamine resistance gene from *Aspergillus oryzae* as a selectable marker of transformed filamentous fungal host cells, and wherein said pyrithiamine resistance gene from *Aspergillus oryzae* has a sequence selected among anyone of the following sequences:
a) the sequence SEQ ID N°4,
b) a sequence having at least 70 % of identity with SEQ ID N°4 encoding a protein which retains the resistance activity to the pyrithiamine,
c) a sequence complementary to anyone of the sequences defined in a) and b).

6. The nucleic acid according to anyone of claims 1 to 5, which comprises a sequence selected among anyone of the following sequences:
a) the sequence SEQ ID N° 5 corresponding to the firefly *Photinus pyralis* luciferase which is under the control of the glyceraldehyde-3-phosphate dehydrogenase promoter from *Aspergillus fumigatus,* and to the pyrithiamine resistance gene from *Aspergillus oryzae* upstream to the promoter,
b) a sequence having at least 70 % of identity with SEQ ID N°5 which retains the activity of the glyceraldehyde-3-phosphate dehydrogenase promoter from *Aspergillus fumigatus,* which encodes a protein which retains the activity of the firefly *Photinus pyralis* luciferase, and which further encodes a protein which retains the resistance activity to the pyrithiamine, and
c) a sequence complementary to anyone of the sequences defined in a) and b).

7. A vector comprising the nucleic acid according to anyone of claims 1 to 6.

8. A transformed filamentous fungal host cell, advantageously a transformed *Aspergillus fumigatus* host cell, comprising the nucleic acid according to anyone of claims 1 to 6 or the vector according to claim 7.

9. The transformed filamentous fungal host cell according to claim 8, wherein said host cell is a transformed *Aspergillus fumigatus* host cell which has been deposited at the CNCM on February 27, 2008 and has the accession number CNCM I- I-3928.

10. An animal model, advantageously a murine model, which has been infected with the transformed filamentous fungal host cell according to claim 8 or 9, said animal model being advantageously an immunosuppressed animal model.

11. A method for determining the virulence of a filamentous fungal host cell, advantageously an *Aspergillus fumigatus* host cell, which comprises the following steps:
a) the transformation of the filamentous fungal host cell with the nucleic acid according to anyone of claims 1 to 6 or with the vector according to claim 7,
b) the culture of the transformed filamentous fungal host cell in order to obtain a transformed filamentous fungal host cell population,
c) the addition to the culture of luciferin which is capable to induce bioluminescence upon reaction under appropriate conditions with the luciferase expressed by the transformed filamentous fungal host cell population,
d) the *in vitro* detection of the emitted bioluminescence, whereby said detection is made advantageously immediately after the step (c) of addition of luciferin, and
e) the determination that the filamentous fungal host cell population is *in vivo* virulent when bioluminescence is detected.

12. Use of the transformed filamentous fungal host cell according to claim 8 or 9 for studying a fungal infection, advantageously for studying specific host immune response mechanisms in response to a filamentous fungal infection.

13. Use of the animal model according to claim 10 for the monitoring of the time-dependent progression of a fungal infection, advantageously for studying specific host immune response mechanisms in response to a filamentous fungal infection.

14. A method for determining the antifungal activity of a compound which comprises the following steps:
a) the transformation of the filamentous fungal host cell with the nucleic acid according to anyone of claims 1 to 6 or with the vector according to claim 7,
b) the culture of the transformed filamentous fungal host cell in order to obtain a transformed filamentous fungal host cell population,
c) the addition to the culture of the compound whose antifungal activity is to be determined,
d) the addition to the culture of luciferin which is capable to induce bioluminescence upon reaction under appropriate conditions with the luciferase expressed by the transformed filamentous fungal host cell population,
e) the quantitative measurement of the emitted bioluminescence, whereby said quantitative measurement is made advantageously immediately after the step (d) of addition of luciferin, and
f) the comparison of the measured quantity of emitted bioluminescence obtained in step (e) with that obtained for the same filamentous fungal host cell whithout addition of the compound whose antifungal activity is to be determined, and
g) the determination that the compound has antifungal activity when the measured quantity of emitted bioluminescence obtained in step (e) is inferior to that obtained for the same filamentous fungal host cell whithout addition of the compound.

15. A method for determining the antifungal activity of a compound which comprises the following steps:
a) the administration of the compound whose antifungal activity is to be determined, to an animal model according to claim 10,
b) the administration of luciferin which is capable to induce bioluminescence upon reaction under appropriate conditions with the luciferase expressed by the transformed filamentous fungal host strain, to the animal model,
c) the quantitative measurement of the emitted bioluminescence in at least one organ of the animal model, whereby said quantitative measurement is made advantageously immediately after the step (b) of administration of luciferin, and
d) the comparison of the measured quantity of emitted bioluminescence obtained in step (c) with that obtained in the organ for the same filamentous fungal host cell whithout addition of the compound whose antifungal activity is to be determined, and
e) the determination that the compound has antifungal activity when the measured quantity of emitted bioluminescence obtained in step (c) is inferior to that obtained for the same filamentous fungal host cell whithout addition of the compound.

16. The method for determining the antifungal activity of a compound according to claim 15, which comprises an additional step (f) of monitoring the health status of the animal model when said model has been infected with a transformed *Aspergillus* host cell, advantageously by monitoring a variation of the body weight and/or the mobility of the animal model.


SEQUENCE LISTING

<110> INSTITUT PASTEUR
LEIBNIZ INSTITUTE FOR NATURAL PRODUCT RESEARCH AND
INFECTION BIOLOGY

<120> Bioluminescent fungi and their application in the study of fungal
infections

<130> D26403

<160> 23

<170> PatentIn version 3.5

<210> 1
<211> 983
<212> DNA
<213> Aspergillus fumigatus

<220>
<223> Promoter glyceraldehyde-3-phosphate dehydrogenase A. fumigatus

<400> 1
gcggccgcag attctagaag tcctgaatag tagtttgtgg attaacattg ttccgatgta 60

ggaatcatga tcccaaccag aagagctgga cagcccctct tccagagcat ttttggtggg 120

atgttttggc ttagtgcgat gcaactggac aaagtccttc cgtttctact gcgtcttaca 180

tcatctggta tctacgcaag ccgcccactt accatatgaa taagaggcac tcaggttttc 240

cctcaccccc ccgaagcgat ggtaagcggg tgccaaatgc atcgggagtt tctctatcat 300

aataacctag gtattccgta atctattacc agtctttccg aagagctggt agcaactgca 360

cgagatttgt aggagcgagt acccggctgg acgagcacgc agcacggcta ttggtcagca 420

tggtagctac cgaggggagg caggccgccc aaatatcgtg agtctcctgc tttgcccggt 480

gtatgaaacc ggaaaagctg ctatagagct tctgggcggc gcatgtcggg aaaccagcag 540

caagctgacc cagaaagacc cgtcctcaag ccattaccgt actaatcaat tatttgtgta 600

gcaacactgg gaagctgtag tgcataggct ggagcagcta tttggccttt agccccgtct 660

gtccgcccgg tgtgcggttt cgactggcgc gcaagctcaa ggtgatcagg tcgttgcgtc 720

agtcggagac aacaagccat tgccttttct actgcccctc ccccgctggt ggcctttttc 780

tctcatcttc tcctctcttc ccatcatcag catcattaat ctactgtctc tctttctttc 840

tatcattcta taaagtaaga acatatccat cttccctcaa tcccgtctac aatagtgtcc 900

tcttcactac tctgtctcta tctctcaaag cttgactgac atttaccccg ctcagtacca 960

gacgaatcta cacaatgaga tct 983

<210> 2

<211>  1657
<212>  DNA
<213>  photinus pyralis

<220>
<223>  Firefly photinus pyralis luciferase gene

<400>  2
agatctgacg ccaaaaacat aaagaaaggc ccggcgccat tctatccgct ggaagatgga      60

accgctggag agcaactgca taaggctatg aagagatacg ccctggttcc tggaacaatt     120

gcttttacag atgcacatat cgaggtggac atcacttacg ctgagtactt cgaaatgtcc     180

gttcggttgg cagaagctat gaaacgatat gggctgaata caaatcacag aatcgtcgta     240

tgcagtgaaa actctcttca attctttatg ccggtgttgg gcgcgttatt tatcggagtt     300

gcagttgcgc ccgcgaacga catttataat gaacgtgaat tgctcaacag tatgggcatt     360

tcgcagccta ccgtggtgtt cgtttccaaa aaggggttgc aaaaaatttt gaacgtgcaa     420

aaaaagctcc caatcatcca aaaaattatt atcatggatt ctaaaacgga ttaccaggga     480

tttcagtcga tgtacacgtt cgtcacatct catctacctc ccggttttaa tgaatacgat     540

tttgtgccag agtccttcga tagggacaag acaattgcac tgatcatgaa ctcctctgga     600

tctactggtc tgcctaaagg tgtcgctctg cctcatagaa ctgcctgcgt gagattctcg     660

catgccagag atcctatttt tggcaatcaa atcattccgg atactgcgat tttaagtgtt     720

gttccattcc atcacggttt tggaatgttt actacactcg gatatttgat atgtggattt     780

cgagtcgtct taatgtatag atttgaagaa gagctgtttc tgaggagcct tcaggattac     840

aagattcaaa gtgcgctgct ggtgccaacc ctattctcct tcttcgccaa aagcactctg     900

attgacaaat acgatttatc taatttacac gaaattgctt ctggtggcgc tcccctctct     960

aaggaagtcg gggaagcggt tgccaagagg ttccatctgc caggtatcag gcaaggatat    1020

gggctcactg agactacatc agctattctg attacacccg agggggatga taaaccgggc    1080

gcggtcggta aagttgttcc attttttgaa gcgaaggttg tggatctgga taccgggaaa    1140

acgctgggcg ttaatcaaag aggcgaactg tgtgtgagag gtcctatgat tatgtccggt    1200

tatgtaaaca atccggaagc gaccaacgcc ttgattgaca aggatggatg gctacattct    1260

ggagacatag cttactggga cgaagacgaa cacttcttca tcgttgaccg cctgaagtct    1320

ctgattaagt acaaaggcta tcaggtggct cccgctgaat tggaatccat cttgctccaa    1380

caccccaaca tcttcgacgc aggtgtcgca ggtcttcccg acgatgacgc cggtgaactt    1440

cccgccgccg ttgttgtttt ggagcacgga aagacgatga cggaaaaaga gatcgtggat    1500

tacgtcgcca gtcaagtaac aaccgcgaaa aagttgcgcg gaggagttgt gtttgtggac    1560

gaagtaccga aaggtcttac cggaaaactc gacgcaagaa aaatcagaga gatcctcata     1620

aaggccaaga agggcggaaa gatcgccgtg taagctt                              1657

<210>  3
<211>  2634
<212>  DNA
<213>  artificial sequence

<220>
<223>  Firefly Photinus pyralis luciferase gene under the control of the
       promoter of the glyceraldehyde-3-phosphate dehydrogenase from
       Aspergillus fumigatus

<400>  3
gcggccgcag attctagaag tcctgaatag tagtttgtgg attaacattg ttccgatgta       60

ggaatcatga tcccaaccag aagagctgga cagcccctct tccagagcat ttttggtggg      120

atgttttggc ttagtgcgat gcaactggac aaagtccttc cgtttctact gcgtcttaca      180

tcatctggta tctacgcaag ccgcccactt accatatgaa taagaggcac tcaggttttc      240

cctcaccccc ccgaagcgat ggtaagcggg tgccaaatgc atcgggagtt tctctatcat      300

aataacctag gtattccgta atctattacc agtctttccg aagagctggt agcaactgca      360

cgagatttgt aggagcgagt acccggctgg acgagcacgc agcacggcta ttggtcagca      420

tggtagctac cgaggggagg caggccgccc aaatatcgtg agtctcctgc tttgcccggt      480

gtatgaaacc ggaaaagctg ctatagagct tctgggcggc gcatgtcggg aaaccagcag      540

caagctgacc cagaaagacc cgtcctcaag ccattaccgt actaatcaat tatttgtgta      600

gcaacactgg gaagctgtag tgcataggct ggagcagcta tttggccttt agccccgtct      660

gtccgcccgg tgtgcggttt cgactggcgc gcaagctcaa ggtgatcagg tcgttgcgtc      720

agtcggagac aacaagccat tgccttttct actgcccctc ccccgctggt ggcctttttc      780

tctcatcttc tcctctcttc ccatcatcag catcattaat ctactgtctc tctttctttc      840

tatcattcta taaagtaaga acatatccat cttccctcaa tcccgtctac aatagtgtcc      900

tcttcactac tctgtctcta tctctcaaag cttgactgac atttaccccg ctcagtacca      960

gacgaatcta cacaatgaga tctgacgcca aaaacataaa gaaaggcccg gcgccattct     1020

atccgctgga agatggaacc gctggagagc aactgcataa ggctatgaag agatacgccc     1080

tggttcctgg aacaattgct tttacagatg cacatatcga ggtggacatc acttacgctg     1140

agtacttcga aatgtccgtt cggttggcag aagctatgaa acgatatggg ctgaatacaa     1200

atcacagaat cgtcgtatgc agtgaaaact ctcttcaatt ctttatgccg gtgttgggcg     1260

cgttatttat cggagttgca gttgcgcccg cgaacgacat ttataatgaa cgtgaattgc     1320

tcaacagtat gggcatttcg cagcctaccg tggtgttcgt ttccaaaaag gggttgcaaa     1380

aaattttgaa cgtgcaaaaa aagctcccaa tcatccaaaa aattattatc atggattcta 1440

aaacggatta ccagggattt cagtcgatgt acacgttcgt cacatctcat ctacctcccg 1500

gttttaatga atacgatttt gtgccagagt ccttcgatag ggacaagaca attgcactga 1560

tcatgaactc ctctggatct actggtctgc ctaaaggtgt cgctctgcct catagaactg 1620

cctgcgtgag attctcgcat gccagagatc ctatttttgg caatcaaatc attccggata 1680

ctgcgatttt aagtgttgtt ccattccatc acggttttgg aatgtttact acactcggat 1740

atttgatatg tggatttcga gtcgtcttaa tgtatagatt tgaagaagag ctgtttctga 1800

ggagccttca ggattacaag attcaaagtg cgctgctggt gccaacccta ttctccttct 1860

tcgccaaaag cactctgatt gacaaatacg atttatctaa tttacacgaa attgcttctg 1920

gtggcgctcc cctctctaag gaagtcgggg aagcggttgc caagaggttc catctgccag 1980

gtatcaggca aggatatggg ctcactgaga ctacatcagc tattctgatt acacccgagg 2040

gggatgataa accgggcgcg gtcggtaaag ttgttccatt ttttgaagcg aaggttgtgg 2100

atctggatac cgggaaaacg ctgggcgtta atcaaagagg cgaactgtgt gtgagaggtc 2160

ctatgattat gtccggttat gtaaacaatc cggaagcgac caacgccttg attgacaagg 2220

atggatggct acattctgga gacatagctt actgggacga agacgaacac ttcttcatcg 2280

ttgaccgcct gaagtctctg attaagtaca aaggctatca ggtggctccc gctgaattgg 2340

aatccatctt gctccaacac cccaacatct tcgacgcagg tgtcgcaggt cttcccgacg 2400

atgacgccgg tgaacttccc gccgccgttg ttgttttgga gcacggaaag acgatgacgg 2460

aaaaagagat cgtggattac gtcgccagtc aagtaacaac cgcgaaaaag ttgcgcggag 2520

gagttgtgtt tgtggacgaa gtaccgaaag gtcttaccgg aaaactcgac gcaagaaaaa 2580

tcagagagat cctcataaag gccaagaagg gcggaaagat cgccgtgtaa gctt 2634

<210> 4
<211> 1982
<212> DNA
<213> Aspergillus oryzae

<220>
<223> Pyrithiamine resistance cassette

<400> 4
gcggccgctt gattacggga tcccattggt aacgaaatgt aaaagctagg agatcgtccg 60

ccgatgtcag gatgatttca cttgtttctt gtccggctca ccggtcaaag ctaaagagga 120

gcaaaaggaa cggatagaat cgggtgccgc tgatctatac ggtatagtgc ccttatcacg 180

ttgactcaac ccatgctatt taactcaacc cctccttctg aaccccacca tcttcttcct 240

tttcctctca tcccacacaa ttctctatct cagatttgaa ttccaaaagt cctcggacga 300

aactgaacaa gtcttcctcc cttcgataaa cctttggtga ttggaataac tgaccatctt 360

ctatagttcc caaaccaacc gacaatgtaa atacactcct cgattagccc tctagagggc 420

atacgatgga agtcatggaa tacttttggc tggactctca caatgatcaa ggtatcttag 480

gtaacgtctt tggcgtgggc cggtgttcgt tcccagtcat cgatgcattc acatgccctc 540

cctaagctgg gccctagact ctaggatcct agtctagaag gacatggcat cgatggactg 600

ggttcgttct gagattatac ggctaaaact tgatctggat aataccagcg aaaagggtca 660

tgccttctct cgttcttcct gttgatggaa tggctaacag atgatagtca ttgcaacttg 720

aaacatgtct cctccagctg ccatctacga acccactgtg gccgctaccg gcctcaaggg 780

taaggtcgtg gtttctgaga ccgtccccgt tgagggagct tctcagacca agctgttgga 840

ccatttcggt ggcaagtggg acgagttcaa gttcgcccct atccgcgaaa gccaggtctc 900

tcgtgccatg accagacgtt actttgagga cctggacaag tacgctgaaa gtgacgttgt 960

cattgttggt gctggttcct gcggtctgag cactgcgtac gtcttggcca aggctcgtcc 1020

ggacctgaag attgctatcg tcgaggccag cgtctctcct ggtcagtagt ccatgatgga 1080

ttgccttgca ctcagctttc cggaactaac gtgcaatagg tggcggtgcc tggttgggtg 1140

gccaactctt ttctgctatg gtcatgcgcc gtcccgcgga agtcttcctg aacgagctgg 1200

gtgttcctta cgaagaggac gcaaacccca actacgttgt cgtcaagcac gcctccctgt 1260

ttacctcgac actcatgtcg aaggttctct ccttccccaa tgtcaagctc ttcaatgcta 1320

ccgctgttga ggacttgatc acccgtccga ccgagaacgg caacccccag attgctggtg 1380

ttgtcgtcaa ctggacgctg gtcacccttc accacgatga tcactcctgc atggacccca 1440

acactatcaa cgctcctgtc atcatcagta ccactggtca cgatgggcca ttcggcgcct 1500

tctgtgcgaa gcgcttggtg tccatgggca gcgtcgacaa gctaggtggc atgcgtggtc 1560

tcgacatgaa ctcggccgag gatgccatcg tcaagaacac ccgcgaggtt actaagggct 1620

tgataatcgg cggtatggag ctgtctgaaa ttgatggctt taaccgcatg ggccctacct 1680

tcggtgccat ggttctcagt ggtgtcaagg ctgccgagga ggcattgaag gtgttcgacg 1740

agcgtcagcg cgagtgtgct gagtaaatga ctcactaccc gaatgggttc agtgcatgaa 1800

ccggatttgt cttacggtct ttgacgatag gggaatgatg attatgtgat agttctgaga 1860

tttgaatgaa ctcgttagct cgtaatccac atgcatatgt aaatggctgt gtcccgtatg 1920

taacggtggg gcattctaga ataattatgt gtaacaagaa agacagtata atacgcggcc 1980

gc 1982

<210>  5
<211>  4608
<212>  DNA
<213>  artificial sequence

<220>
<223>  Firefly Photinus pyralis luciferase gene under the control of the
       promoter of the glyceraldehyde-3-phosphate dehydrogenase from
       Aspergillus fumigatus and pyrithiamine resistance cassette

<400>  5
gcggccgctt gattacggga tcccattggt aacgaaatgt aaaagctagg agatcgtccg      60

ccgatgtcag gatgatttca cttgtttctt gtccggctca ccggtcaaag ctaaagagga     120

gcaaaaggaa cggatagaat cgggtgccgc tgatctatac ggtatagtgc ccttatcacg     180

ttgactcaac ccatgctatt taactcaacc cctccttctg aaccccacca tcttcttcct     240

tttcctctca tcccacacaa ttctctatct cagatttgaa ttccaaaagt cctcggacga     300

aactgaacaa gtcttcctcc cttcgataaa cctttggtga ttggaataac tgaccatctt     360

ctatagttcc caaaccaacc gacaatgtaa atacactcct cgattagccc tctagagggc     420

atacgatgga agtcatggaa tacttttggc tggactctca caatgatcaa ggtatcttag     480

gtaacgtctt tggcgtgggc cggtgttcgt tcccagtcat cgatgcattc acatgccctc     540

cctaagctgg gccctagact ctaggatcct agtctagaag gacatggcat cgatggactg     600

ggttcgttct gagattatac ggctaaaact tgatctggat aataccagcg aaaagggtca     660

tgccttctct cgttcttcct gttgatggaa tggctaacag atgatagtca ttgcaacttg     720

aaacatgtct cctccagctg ccatctacga acccactgtg gccgctaccg gcctcaaggg     780

taaggtcgtg gtttctgaga ccgtccccgt tgagggagct tctcagacca agctgttgga     840

ccatttcggt ggcaagtggg acgagttcaa gttcgcccct atccgcgaaa gccaggtctc     900

tcgtgccatg accagacgtt actttgagga cctggacaag tacgctgaaa gtgacgttgt     960

cattgttggt gctggttcct gcggtctgag cactgcgtac gtcttggcca aggctcgtcc    1020

ggacctgaag attgctatcg tcgaggccag cgtctctcct ggtcagtagt ccatgatgga    1080

ttgccttgca ctcagctttc cggaactaac gtgcaatagg tggcggtgcc tggttgggtg    1140

gccaactctt ttctgctatg gtcatgcgcc gtcccgcgga agtcttcctg aacgagctgg    1200

gtgttcctta cgaagaggac gcaaacccca actacgttgt cgtcaagcac gcctccctgt    1260

ttacctcgac actcatgtcg aaggttctct ccttccccaa tgtcaagctc ttcaatgcta    1320

ccgctgttga ggacttgatc acccgtccga ccgagaacgg caacccccag attgctggtg    1380

ttgtcgtcaa ctggacgctg gtcacccttc accacgatga tcactcctgc atggacccca    1440

acactatcaa cgctcctgtc atcatcagta ccactggtca cgatgggcca ttcggcgcct    1500

tctgtgcgaa gcgcttggtg tccatgggca gcgtcgacaa gctaggtggc atgcgtggtc 1560

tcgacatgaa ctcggccgag gatgccatcg tcaagaacac ccgcgaggtt actaagggct 1620

tgataatcgg cggtatggag ctgtctgaaa ttgatggctt taaccgcatg ggccctacct 1680

tcggtgccat ggttctcagt ggtgtcaagg ctgccgagga ggcattgaag gtgttcgacg 1740

agcgtcagcg cgagtgtgct gagtaaatga ctcactaccc gaatgggttc agtgcatgaa 1800

ccggatttgt cttacggtct ttgacgatag gggaatgatg attatgtgat agttctgaga 1860

tttgaatgaa ctcgttagct cgtaatccac atgcatatgt aaatggctgt gtcccgtatg 1920

taacggtggg gcattctaga ataattatgt gtaacaagaa agacagtata atacgcggcc 1980

gcagattcta gaagtcctga atagtagttt gtggattaac attgttccga tgtaggaatc 2040

atgatcccaa ccagaagagc tggacagccc ctcttccaga gcatttttgg tgggatgttt 2100

tggcttagtg cgatgcaact ggacaaagtc cttccgtttc tactgcgtct tacatcatct 2160

ggtatctacg caagccgccc acttaccata tgaataagag gcactcaggt tttccctcac 2220

ccccccgaag cgatggtaag cgggtgccaa atgcatcggg agtttctcta tcataataac 2280

ctaggtattc cgtaatctat taccagtctt tccgaagagc tggtagcaac tgcacgagat 2340

ttgtaggagc gagtacccgg ctggacgagc acgcagcacg gctattggtc agcatggtag 2400

ctaccgaggg gaggcaggcc gcccaaatat cgtgagtctc ctgctttgcc cggtgtatga 2460

aaccggaaaa gctgctatag agcttctggg cggcgcatgt cgggaaacca gcagcaagct 2520

gacccagaaa gacccgtcct caagccatta ccgtactaat caattatttg tgtagcaaca 2580

ctgggaagct gtagtgcata ggctggagca gctatttggc ctttagcccc gtctgtccgc 2640

ccggtgtgcg gtttcgactg gcgcgcaagc tcaaggtgat caggtcgttg cgtcagtcgg 2700

agacaacaag ccattgcctt ttctactgcc cctcccccgc tggtggcctt tttctctcat 2760

cttctcctct cttcccatca tcagcatcat taatctactg tctctctttc tttctatcat 2820

tctataaagt aagaacatat ccatcttccc tcaatcccgt ctacaatagt gtcctcttca 2880

ctactctgtc tctatctctc aaagcttgac tgacatttac cccgctcagt accagacgaa 2940

tctacacaat gagatctgac gccaaaaaca taaagaaagg cccggcgcca ttctatccgc 3000

tggaagatgg aaccgctgga gagcaactgc ataaggctat gaagagatac gccctggttc 3060

ctggaacaat tgcttttaca gatgcacata tcgaggtgga catcacttac gctgagtact 3120

tcgaaatgtc cgttcggttg gcagaagcta tgaaacgata tgggctgaat acaaatcaca 3180

gaatcgtcgt atgcagtgaa aactctcttc aattctttat gccggtgttg ggcgcgttat 3240

ttatcggagt tgcagttgcg cccgcgaacg acatttataa tgaacgtgaa ttgctcaaca 3300

gtatgggcat ttcgcagcct accgtggtgt tcgtttccaa aaaggggttg caaaaaattt 3360

tgaacgtgca aaaaaagctc ccaatcatcc aaaaaattat tatcatggat tctaaaacgg 3420

attaccaggg atttcagtcg atgtacacgt tcgtcacatc tcatctacct cccggtttta 3480

atgaatacga ttttgtgcca gagtccttcg atagggacaa gacaattgca ctgatcatga 3540

actcctctgg atctactggt ctgcctaaag gtgtcgctct gcctcataga actgcctgcg 3600

tgagattctc gcatgccaga gatcctattt ttggcaatca aatcattccg gatactgcga 3660

ttttaagtgt tgttccattc catcacggtt ttggaatgtt tactacactc ggatatttga 3720

tatgtggatt tcgagtcgtc ttaatgtata gatttgaaga agagctgttt ctgaggagcc 3780

ttcaggatta caagattcaa agtgcgctgc tggtgccaac cctattctcc ttcttcgcca 3840

aaagcactct gattgacaaa tacgatttat ctaatttaca cgaaattgct tctggtggcg 3900

ctcccctctc taaggaagtc ggggaagcgg ttgccaagag gttccatctg ccaggtatca 3960

ggcaaggata tgggctcact gagactacat cagctattct gattacaccc gagggggatg 4020

ataaaccggg cgcggtcggt aaagttgttc cattttttga agcgaaggtt gtggatctgg 4080

ataccgggaa aacgctgggc gttaatcaaa gaggcgaact gtgtgtgaga ggtcctatga 4140

ttatgtccgg ttatgtaaac aatccggaag cgaccaacgc cttgattgac aaggatggat 4200

ggctacattc tggagacata gcttactggg acgaagacga acacttcttc atcgttgacc 4260

gcctgaagtc tctgattaag tacaaaggct atcaggtggc tcccgctgaa ttggaatcca 4320

tcttgctcca acaccccaac atcttcgacg caggtgtcgc aggtcttccc gacgatgacg 4380

ccggtgaact tcccgccgcc gttgttgttt tggagcacgg aaagacgatg acggaaaaag 4440

agatcgtgga ttacgtcgcc agtcaagtaa caaccgcgaa aaagttgcgc ggaggagttg 4500

tgtttgtgga cgaagtaccg aaaggtctta ccggaaaact cgacgcaaga aaaatcagag 4560

agatcctcat aaaggccaag aagggcggaa agatcgccgt gtaagctt 4608

<210> 6
<211> 29
<212> DNA
<213> artificial sequence

<220>
<223> Primer LucBgl_up

<400> 6
agatctgacg ccaaaaacat aaagaaagg 29

<210> 7
<211> 24
<212> DNA
<213> artificial sequence

<220>
<223>  Primer LucHind_down

<400>  7
aagcttacac ggcgatcttt ccgc                                              24


<210>  8
<211>  20
<212>  DNA
<213>  artificial sequence

<220>
<223>  Primer LucMi_up

<400>  8
gatgtccacc tcgatatgtg                                                   20


<210>  9
<211>  25
<212>  DNA
<213>  artificial sequence

<220>
<223>  Primer PgpdAAfNot_up

<400>  9
gcggccgcag attctagaag tcctg                                             25


<210>  10
<211>  29
<212>  DNA
<213>  artificial sequence

<220>
<223>  Primer PgpdAAfBglATG

<400>  10
agatctcatt gtgtagattc gtctggtac                                         29


<210>  11
<211>  29
<212>  DNA
<213>  artificial sequence

<220>
<223>  Primer Not_ptrA_up_for

<400>  11
gcggccgctt gattacggga tcccattgg                                         29


<210>  12
<211>  33
<212>  DNA
<213>  artificial sequence

<220>
<223>  Primer Not_ptrA_dow_rev

<400>  12
gcggccgcgt attatactgt ctttcttgtt aca                                    33


<210>  13
<211>  23
<212>  DNA
<213>  artificial sequence

<220>
<223>  Primer BglAfGPDcDNA_up

<400>  13
agatctgcca ctcccaaggt tgg                                               23


<210>  14
<211>  24
<212>  DNA
<213>  artificial sequence

<220>
<223>  Primer HindAfGPDcDNA_do

<400>  14
aagcttactg ggaatcgacc ttgg                                              24


<210>  15
<211>  19
<212>  DNA
<213>  artificial sequence

<220>
<223>  Primer cDNAgpdAAf_up

<400>  15
cgtcgatgct ccctccaac                                                    19


<210>  16
<211>  19
<212>  DNA
<213>  artificial sequence

<220>
<223>  Primer cDNAgpdAAf_do

<400>  16
ggaatcgacc ttggcgatg                                                    19


<210>  17
<211>  19
<212>  DNA
<213>  artificial sequence

<220>

<223>  Primer lucProbe_up

<400>  17
gatggaaccg ctggagagc                                                    19


<210>  18
<211>  23
<212>  DNA
<213>  artificial sequence

<220>
<223>  Primer lucProbe_mi_rev

<400>  18
ccaaaaccgt gatggaatgg aac                                               23


<210>  19
<211>  18
<212>  DNA
<213>  artificial sequence

<220>
<223>  Primer AfCitAcode_up

<400>  19
gcaaggtcat cggcgagg                                                     18


<210>  20
<211>  19
<212>  DNA
<213>  artificial sequence

<220>
<223>  Primer AfCitAcode_do

<400>  20
gttgtcaccg tagccgagc                                                    19


<210>  21
<211>  20
<212>  DNA
<213>  artificial sequence

<220>
<223>  Primer act_Afum_for_II

<400>  21
gctttgctac gtcgctctcg                                                   20


<210>  22
<211>  21
<212>  DNA
<213>  artificial sequence

<220>
<223>  Primer act_Afum_rev_II

<400>  22
ggaggagcaa tgatcttgac c                                                21

<210>  23
<211>  22
<212>  DNA
<213>  artificial sequence

<220>
<223>  Primer anchored Oligo(dT)20

<220>
<221>  misc_feature
<222>  (1)..()
<223>  n is A, T, C or G

<220>
<221>  misc_feature
<222>  (2)..()
<223>  n is A, C or G

<400>  23
nntttttttt tttttttttt tt                                               22